Europäisches Patentamt

European Patent Office

Office européen des brevets

(19)

(11) Veröffentlichungsnummer: **0 084 522**
**A2**

(12) **EUROPÄISCHE PATENTANMELDUNG**

(21) Anmeldenummer: 83810014.7

(22) Anmeldetag: 13.01.83

(51) Int. Cl.³: **C 12 N 15/00**
**A 61 K 39/108**

(30) Priorität: 18.01.82 CH 282/82
05.03.82 CH 1362/82
10.03.82 CH 1478/82

(43) Veröffentlichungstag der Anmeldung:
27.07.83 Patentblatt 83/30

(84) Benannte Vertragsstaaten:
AT BE CH DE FR GB IT LI LU NL SE

(71) Anmelder: **SANDOZ AG**
**Lichtstrasse 35**
**CH-4002 Basel(CH)**

(84) Benannte Vertragsstaaten:
BE CH FR GB IT LI LU NL SE

(71) Anmelder: **SANDOZ-PATENT-GMBH**
**Humboldtstrasse 3**
**D-7850 Lörrach(DE)**

(84) Benannte Vertragsstaaten:
**DE**

(71) Anmelder: **SANDOZ-ERFINDUNGEN**
**Verwaltungsgesellschaft m.b.H.**
**Brunner Strasse 59**
**A-1235 Vienna(AT)**

(84) Benannte Vertragsstaaten:
**AT**

(72) Erfinder: **Ceska, Miroslav**
**Habsburgerstrasse 78**
**A-2500 Baden(AT)**

(72) Erfinder: **Kricek, Franz**
**Ulmenweg 9**
**A-2362 Biedermannsdorf(AT)**

(72) Erfinder: **Högenauer, Gregor**
**Wienerbruckstrasse 124/54**
**A-2344 Maria Enzersdorf(AT)**

(72) Erfinder: **Besemer, Jürgen**
**Zwillinggasse 17/2**
**A-2340 Mödling(AT)**

(54) Neue Plasmide und Bakterienstämme sowie Verfahren zu ihrer Herstellung.

(57) Plasmide und Bakterienstämme zur Produktion von Enterotoxoide und Antigene. Insbesondere die Plasmide bzw. Bakterienstämme, pEFK–600, –700, –800 und pEBK 620 : E. coli K12 5K/pEFK-600, –700 + pRK248clts, –800 + pRK248clts und E. coli K12 5K/pEBK–620 : E. coli K12 5K/pEFK–600 + K88(AB), –700 + pRK248clts + K88(AB), –800 + pRK 248clts + K88(AB) und E. coli K12 5K/pEBK–620 + K88(AB,AC,AD). Die erhaltenen Enterotoxoide und/oder Antigene können zur Herstellung von Vakzinen eingesetzt werden.

EP 0 084 522 A2

Neue Plasmide und Bakterienstämme sowie Verfahren zu ihrer Herstellung

Escherichia coli Keime, die im humanen sowie veterinären Bereich choleraähnliche Durchfallserkrankungen hervorrufen, produzieren zwei unterschiedliche Typen von Enterotoxinen. Eines dieser E. coli Enterotoxine ist das hitzelabile Enterotoxin (LT). Die Existenz einer immunologischen Verwandtschaft dieses Toxins mit dem Enterotoxin (Choleragen) aus Vibrio cholerae ist gesichert. Außerdem ist der Wirkungsmechanismus des E. coli Enterotoxins mit dem des Choleratoxins prinzipiell identisch und besteht in einer Aktivierung der Adenylatcyclase, die zu einer Erhöhung des intrazellulären cAMP-Spiegels und damit zu einer vermehrten Flüssigkeitsexkretion in das Darmlumen führt.

Beim E. coli LT-Holotoxin handelt es sich um ein Protein mit einem pI von 8,0 und einer ungefähren relativen Molekülmasse von 91.440. Analog dem Choleragen kann auch LT in 2 Proteinuntereinheiten dissoziieren. Die isolierten Untereinheiten besitzen relative Molekülmassen von 25.000 (Untereinheit A) bzw. 11.500 (Untereinheit B), wobei die große Untereinheit für die toxischen Eigenschaften und die kleinere Untereinheit für den immunogenen Charakter des Toxins verantwortlich sind.

Genetisch wird LT meist von großen, transmissiblen Plasmiden kodiert, wobei über die Regulation der Genexpression noch wenig bekannt ist. Zur vollen Ausbildung ihrer Pathogenität müssen enterotoxinproduzierende E. coli Zellen außerdem noch über einen wirtsspezifischen Adhäsionsfaktor verfügen, der eine ausreichende Kolonisierung im Wirtsdarm ermöglicht. Solche Kolonisationsfaktoren sind ebenfalls plasmidkodiert und wurden in schweine-, rinder- und humanpathogenen Enterotoxinproduzenten nachgewiesen.

Zum Zweck einer wirtschaftlichen Impfstoffproduktion auf Enterotoxinbasis ist es notwendig, eine ausreichende Menge genügend reinen Enterotoxins zu Immunisierungszwecken billig fermentativ herstellen zu können. Weiters ist ein atoxisches, aber immunogenes Produkt von Vorteil. Wird ein solches Protein bereits von geeigneten Bakterien synthetisiert, so können diese direkt als Lebendvakzine eingesetzt werden.

Die bekannten, aus infizierten Schweinen isolierten, LT-produzierenden Bakterienstämme E. coli 711/P307 sowie E. coli P263 erwiesen sich als zu schwache Enterotoxinproduzenten. Die Zellüberstände sind außerdem durch Endotoxin (bakterielle Lipopolysaccharide aus der gramnegativen Zellwand) verunreinigt, so daß eine minimale Enterotoxinkonzentration in den Fermentationsbrühen nicht unterschritten werden darf, um dessen toxische und pyrogene Nebenwirkungen während der Immunisierung in Grenzen zu halten. Bisherige Versuche zur Verbesserung der Enterotoxinausbeute durch Optimierung der Fermentationsbedingungen von Wildtyp-Stämmen führten nicht zum gewünschten Erfolg.

Mit Hilfe der in vitro-Genrekombinationstechnik konnten sowohl Produzentenkeime mit höherer Toxinausbeute wie auch Bakterienklone isoliert werden, die ein immunogenes, aber nicht toxisches Enterotoxoid synthetisieren.

Zunächst wurde das Enterotoxinplasmid P307 aus dem schweinepathogenen E. coli 711 Stamm isoliert und mit Hilfe geeigneter Restriktionsenzyme und Auftrennung der einzelnen Fragmente im Agarosegel charakterisiert. Das Gen für hitzelabiles Enterotoxin befindet sich zur Gänze auf einem BamHI-Bruchstück mit einer relativen Molekülmasse von 5,8 Md (8,8 kb) des Plasmids P307. Dieses DNA-Fragment wurde mit dem Plasmidvektor pBR325 ligiert, der in einer erhöhten Kopienzahl von 25 - 30 pro Zelle vorliegt (Abb. 1).

Nach Selektion auf chloramphenicol- und ampicillinresistente aber tetracyclinsensitive Klone wurde aus allen erhaltenen Rekombinanten in einem analytischen Minilysatverfahren (H.C. Birnboim, J. Doly: Nuc.Ac.Res. 7/1513 [1979]) die Plasmid-DNA isoliert, mit geeigneten Restriktionsendonukleasen gespalten und durch Auftrennung im Agarosegel charakterisiert. Jene beiden Keime, deren Plasmide sich als identisch erwiesen und ein BamHI-Restriktionsfragment von 5,8 Md (8,8 kb) enthielten, wurden auf Enterotoxinproduktion geprüft. Dieses Plasmid wurde mit pEFK295 bezeichnet. Als Test auf gebildetes Ent-LT fand das in der Literatur (D. Evans, D.G. Evans: Infect.Immun.16/604 [1977]) beschriebene passive

Immunhämolyseverfahren Verwendung, das im Prinzip die Bindungsfähigkeit von hitzelabilem Enterotoxin an Schaferythrozyten und die immunologische Kreuzreaktivität von LT mit gegen Choleratoxin gerichteten Antikörpern ausnützt (siehe Tabelle 1).

## TABELLE 1:
## PASSIVER IMMUNOHÄMOLYSETEST (PIH)

Schaferythrocyten
$\mid$—Ent LT
Ery-LT (sensitiv)
$\mid$—Kaninchen-Antiserum
Ery-LT-aLT
$\mid$— Meerschweinchen-Komplement
Ery-LT-aLT-Komp.
$\mid$
Hämolyse

Die praktische Durchführung erfolgt auf speziellen Blutplatten. Man erhält Hämolysehöfe, deren Durchmesser sich als Funktion der Toxinkonzentration mit Hilfe einer Choleratoxin-Eichkurve quantitativ auswerten lassen. Die Aufzucht der Keime zur Probennahme fand ·in einem glucosearmen Minimalmedium unter standardisierten Bedingungen statt, so daß die Bakterienzellen jeweils etwa am Beginn der stationären Wachstumsphase bei einer durchschnittlichen Keimzahl von ungefähr $1 \times 10^8$/ml geerntet wurden. Es zeigte sich, daß in den Lysaten ultrabeschallter Zellen der beiden Klone eine wesentlich·höhere Menge an Enterotoxin nachgewiesen werden konnte, als dies in entsprechenden Zellysaten von Ent-LT Wildtypstämmen der Fall war. Aus den Immunhämolyse-Testergebnissen ergibt sich folgendes Bild (Tabelle 2):

TABELLE 2:

| Keim: | $\mu g\ VCT^{+)}$ / Liter |
|---|---|
| E. coli 711/P307 | 24 ± 38% |
| E. coli P263 | 23 ± 11% |
| E. coli H10407 | 24 ± 31% |
| E. coli K12 5K/ pEFK295 | 1360 ± 50% |

+)μg Choleratoxin-Äquivalente pro 1 Zellkultur.

Der intrazelluläre Anteil an hitzelabilem Enterotoxin liegt damit in den durch Genrekombination hergestellten E. coli Keimen um einen Faktor von etwa 50 höher als im entsprechenden Wildtypstamm E. coli 711/P307.

Eine physikalische Kartierung des Plasmids pEFK295 mit einer Reihe verschiedener Restriktionsendonukleasen zeigte, daß pEFK295 das gesamte Strukturgen für hitzelabiles Enterotoxin trägt. Auch die Orientierung des klonierten BamHI-Fragments konnte mit Hilfe der erstellten Genkarte eindeutig festgelegt werden (Abb. 2). Auf Grund der Tatsache, daß pEFK295 das gesamte Strukturgen für hitzelabiles Enterotoxin besitzt, war anzunehmen, daß das synthetisierte Genprodukt nicht nur die immunogene, sondern auch die volle toxische Wirkung des Wildtyp-Toxins aufweist. Sowohl im Hinblick auf einen eventuellen Einsatz als Lebendvakzine wie auch eine allfällig mögliche großtechnische Fermentation wurde durch ein weiteres Klonierungsexperiment ein Bakterienstamm erhalten, der imstande ist, ein atoxisches, aber immunogenes Enterotoxin in erhöhter Ausbeute zu synthetisieren. Dies gelang durch die enzymatische Öffnung des Plasmids pEFK295 mit der Restriktionsendonuklease EcoRI und anschließenden Ringschluß des größeren der dabei entstandenen Fragmente durch Ligierung mit T4-Ligase. Dabei wurde die dem Enterotoxin-Gen vorgeschaltete Promotorregion entfernt. Korrekte Transkription des verbliebenen Enterotoxingenfragments konnte nur von einem geeigneten Promotor des Vektorplasmids, möglicherweise dem Chloramphenicolacetyltransferase-Promotor, erwartet werden. Die beabsichtigte Deletion im neu entstandenen Plasmid mit der Bezeichnung pEFK500 konnte durch physikalische Kartierung der aus den Chloramphenicol(Cm)-sensitiven Keimen isolierten Plasmid-DNA bestätigt werden (Abb. 3).

pEFK500 tragende E. coli Keime sind im passiven Immunhämolysetest positiv. Die Ausbeuten sind mit jenen der pEFK295 tragenden Keime vergleichbar. Ein Enterotoxintest in Jejunum-Ligaturen von Kaninchen zeigte, daß sich Zellüberstände bzw. -lysate von Keimen mit den Plasmiden P307 sowie pEFK295 als toxisch erwiesen, während Extrakte von pEFK500 tragenden Keimen atoxisch waren. Diese Ergebnisse konnten in einem in vitro Toxizitätstest an Y1-Zellen bestätigt werden: Nur P307- bzw. pEFK295 tragende Keime zeigten die für Enterotoxineinwirkung charakteristischen morphologischen Veränderungen.

Der Enterotoxingehalt aller Keime ist vom Nährmedium abhängig. Durch Zusatz von Ampicillin (Ap) zu pEFK295 bzw. pEFK500 tragenden Bakterienkulturen kann eine zusätzliche Steigerung der Toxinausbeuten erreicht werden (Tabelle 3).

## TABELLE 3:

| Plasmid<br>µg VCT/l | P307 | pEFK295 | pEFK500 |
|---|---|---|---|
| F+AB[1] | --- | 1137±40% | 1082±13% |
| F[2] | 24±38% | 628±28% | 353±60% |
| TSB+AB[3] | --- | 386±10% | 113±9% |
| TSB[4] | 0 | 47±26% | 0 |
| M9+AB[5] | --- | 1640±17% | 24±21% |
| M9[6] | 23±11% | 1644±10% | 50±10% |

1) Finkelstein-Medium + 50 μg/ml Ap

2) Finkelstein-Medium ohne Ap

3) Trypticase Soy Broth + 50 μg/ml Ap

4) Trypticase Soy Broth ohne Ap

5) Synthetisches M9-Medium mit Glycerin als einziger C-Quelle + 50 μg/ml Ap

6) Synthetisches M9-Medium mit Glycerin als einziger C-Quelle ohne Ap.

Die Herstellung eines weiteren, Enterotoxoid-produzierenden Bakterienstammes gelang durch Spaltung des Plasmids pEFK295 sowie des Vektorplasmids pBR322 durch die Restriktionsendonuklease Hind III und Ligierung der entstandenen Bruchstücke mit T4-Ligase.

Nach Selektion sowohl auf chloramphenicol- und ampicillinresistente, aber tetracyclinsensitive, wie auch auf ausschließlich ampicillinresistente Klone wurde aus allen erhaltenen Rekombinanten in einem analytischen Minilysatverfahren (H.C. Birnboim, J.Doly: Nuc.Ac.Res. 7/1513 [1979]) die Plasmid-DNA isoliert, mit geeigneten Restriktionsendonukleasen gespalten und durch Auftrennung im Agarosegel charakterisiert. Jene Keime, deren rekombinante Plasmide Ent-LT Genfragmente enthielten, wurden auf die Synthese von E.coli Enterotoxin bzw. -toxoid, wie bereits beschrieben, getestet.

Von den ausschließlich ampicillinresistenten Klonen erwies sich keiner als im Immunhämolysetest positiv. Der Bakterienklon, welcher sowohl Ampicillin- wie auch Chloramphenicolresistenz aufwies, zeigte positive Hämolysereaktion. Ein anschließend durchgeführter in vitro Toxizitätstest an Y1-Zellen verlief für alle untersuchten Klone negativ.

Eine physikalische Charakterisierung der aus den beschriebenen Bakterienklonen isolierten Plasmide mit den Bezeichungen pEFK502, pEFK600 und pEFK610 durch vergleichende Spaltung mit verschiedenen Restriktionsenzymen führte zu folgendem Ergebnis (Abb.4).

pEFK502 trägt demnach jenen Teil des Ent-LT Gens (a), der für die toxische A-Untereinheit des hitzelabilen E.coli Enterotoxins kodiert, während in pEFK610 das Gen für die bindende B-Untereinheit integriert ist (b). Bei beiden Plasmiden stammt der Vektoranteil von pBR322. Das rekombinante Plasmid pEFK600 enthält ebenfalls nur mehr jenes HindIII-Fragment des Ent-LT Plasmids pEFK295, welches das Gen für die B-Untereinheit trägt. Der Vektoranteil stammt in diesem Fall jedoch gleichfalls aus dem Plasmid pEFK295 und damit vom ursprünglich eingesetzten Plasmidvektor pBR325.

Die Enterotoxoidausbeuten pEFK600 tragender Keime sind entsprechend den Ergebnissen der Immunhämolysetests mit jenen der pEFK295 tragenden Keime vergleichbar (siehe Tabelle 4).

### TABELLE 4:

Durchschnittliche Enterotoxin (-toxoid)-Ausbeuten

| Keim: | Plasmid: | Ent-LT (Ü) 1) [μg/l] | Ent-LT (Z) 2) [μg/l] | Entero-toxin: | Herkunft: |
|---|---|---|---|---|---|
| E.coli 711 | P307 | <10 3) | 25±40% | Toxin | Wildtyp-porcin |
| E.coli K12 5K | pEFK295 | 830±60% | 1360±50% | Toxin | P307 $\xrightarrow{[\text{Bam HI}]}$ pEFK295  pBR325 |
| E.coli K12 5K | pEFK500 | 280±40% | 850±40% | Toxoid | pEFK295 $\xrightarrow{[\text{EcoRI}]}$ → pEFK 500 |
| E.coli K12 5K | pEFK600 | 460±30% | 1430±30% | Toxoid | pEFK295 $\xrightarrow{[\text{HindIII}]}$ → pEFK600 |

1) Enterotoxin (-toxoid)-Konzentration im Kulturüberstand nach Abzentrifugation des Zellmaterials, angegeben in μg Choleratoxin-Äquivalenten pro Zellmasse, gewonnen aus einem Liter Kulturbrühe.

2) Intrazelluläre Enterotoxin (-toxoid)-Menge aus zentrifugiertem, ultrabeschalltem Zellmaterial, angegeben in µg Choleratoxin-Äquivalenten pro Zellmasse, gewonnen aus einem Liter Kulturbrühe.

3) Die Nachweisgrenze des unter den beschriebenen Bedingungen durchgeführten passiven Immunhämolysetests liegt bei etwa 10 µg VCT-Äquivalenten/Liter. In dem mit <10 bezeichneten Fall konnten im Immunhämolysetest keine meßbaren Hämolysehöfe nachgewiesen werden.

Um zu Bakterienklonen, die in hoher Ausbeute ein von Ent-LT abgeleitetes, nicht toxisches aber immunogenes Enterotoxoid synthetisieren, zu gelangen, wurde versucht, durch Rekombinationsexperimente das Strukturgen für die B-Untereinheit des hitzelabilen E.coli Enterotoxins unter die Kontrolle eines bekannt effektiven Promotors zu bringen. Als ein solcher Promotor wurde ein vom Bakteriophagen $\lambda$ abgeleiteter Promotor mit der Bezeichnung $p_L$ eingesetzt. Ein Bakterienplasmid mit der Bezeichnung pHUB2, welches diesen Promotor trägt, wurde von Ulrich Bernard von der Universität San Diego, Kalifornien, USA, zur Verfügung gestellt (Abb. 5) (Bernard et al, Gene 5, 1979, 59-76).

Die Vorschaltung des $p_L$-Promotors vor das Ent-LT-B Gen erfolgte in zwei Schritten. Zunächst wurde durch Spaltung des Plasmids pEFK295 sowie des Vektorplasmids pHUB2 mit dem Restriktionsenzym BamHI und anschließende Ligierung der entstandenen Fragmente ein dem Plasmid pEFK295 analoges rekombinantes Plasmid mit der Bezeichnung pEFK700 hergestellt (Abb. 6).

Um die $p_L$-Promotorregion näher an das Enterotoxin-Gen heranzubringen und gleichzeitig das Strukturgen für die toxische Entertoxinuntereinheit zu entfernen, wurde das Plasmid pEFK700 mit der Restriktionsendonuklease EcoR1 gespalten und die entstandenen Bruchstücke unter geeigneten Bedingungen religiert. Nach Transformation von Bakterien und Selektion geeigneter Rekombinanten mit Hilfe des analytischen Minilysatverfahrens konnten Keime isoliert werden, die ein vom Plasmid pEFK700 abgeleitetes, entsprechend verkürztes Plasmid mit der Bezeichnung pEFK800 enthielten. Dieses Plasmid enthält die Ent-LT-B Genregion, der der bekannt effektive $\lambda$-Promotor vorgeschaltet ist (Abb. 7).

Da aus der Literatur bekannt ist (Bernard et al, Gene 5, 1979, 59-76), daß Plasmide, die den aktiven $p_L$-Promotor enthalten, genetisch instabil sind, wurden als Wirtsbakterien für alle Plasmide, die diesen Promotor tragen, E.coli K12 5K Keime verwendet, die bereits ein weiteres Plasmid mit der Bezeichnung pRK248cIts enthielten. Dieses Plasmid stammt ebenfalls von Ulrich Bernard und trägt das Gen für ein mit cI bezeichnetes Repressorprotein, welches seinerseits den $\lambda$-Promotor $p_L$ negativ reguliert. Ist dieses Repressorprotein cI in der Zelle vorhanden, so bindet es an die $p_L$-Promotorregion im Operatorbereich und schaltet somit diesen Promotor und alle nachfolgenden Strukturgene ab. Das Plasmid pRK248cIts trägt eine temperatursensitive Mutante dieser cI Repressorgenregion, was bedeutet, daß das cI Repressorprotein nur bei Temperaturen von etwa 30° C, nicht jedoch bei Temperaturen von mehr als 37° C synthetisiert wird. Dadurch ist es möglich, etwa in Fermentationsbrühen durch Einstellen einer bestimmten Temperatur den $\lambda$-Promotor gezielt an- bzw. abzuschalten. Es ist somit möglich, durch Wachstum bei niederer Temperatur zu einer hohen Zelldichte plasmidtragender Keime zu kommen und anschließend durch Temperaturerhöhung für mehrere Stunden die so angezüchteten Bakterien zur Expression des Ent-LT-B Genprodukts zu zwingen.

Nach Transformation der beschriebenen Wirtsbakterien mit den entsprechenden, nach Restriktionsspaltung und Ligierung mit T4-Ligase entstandenen DNA-Gemischen wurde auf kanamycin- und tetracyclinresistente Klone selektioniert. Aus allen erhaltenen Rekombinanten wurde im analytischen Minilysatverfahren die Plasmid-DNA isoliert, mit geeigneten Restriktionsendonukleasen gespalten und durch Auftrennung im Agarosegel charakterisiert. Jene beiden Klone, welche die in Abb. 6 und Abb. 7 dargestellten rekombinanten Plasmide beherbergen, wurden auf Enterotoxinproduktion geprüft. Als Test auf gebildetes Ent-LT fand das bereits erwähnte passive Immunhämolyseverfahren Verwendung.

Die mit den Bakterienklonen, welche die Ent-Plasmide pEFK700 bzw. pEFK800 beherbergen, unter den gewählten Kulturbedingungen erreichbaren Enterotoxin- bzw. Enterotoxoidausbeuten sind in der Tabelle 5 zusammengefaßt. Verglichen mit dem Wildtypstamm E.coli 711/P307 beträgt die Steigerung der Enterotoxin- bzw. Enterotoxoidausbeuten somit das 80 bis 100-fache im Fall des Plasmids pEFK700 und das 200 bis 250-fache im Fall des Plasmids pEFK 800.

Die zu erwartende Abhängigkeit der Ausbeute von den Züchtungsbedingungen, je nachdem, ob der $p_L$-Promotor durch das cI Genprodukt des Plasmids pRK248cIts an- oder abgeschaltet ist, trat nur bei Keimen mit dem Plasmid pEFK800 ausgeprägt zutage (Tabelle 6).

Aufgrund der Entfernung der $p_L$-Promotorregion vom Ent-LT Strukturgen im Plasmid pEFK700 scheint dieser Promotor auf die Expression des Enterotoxin-Gens wenig Einfluß auszuüben. Die Transkription des Ent-LT Gens erfolgt wahrscheinlich vornehmlich vom eigenen Ent-LT Promotor aus (Abb. 6).

Im Plasmid pEFK800 fehlt der Ent-LT Promotor, und die $p_L$-Promotorsequenz liegt direkt vor dem Ent-LT-B Strukturgen (Abb. 7). Aus der Aktivität des $p_L$-Protomors resultiert die von den Züchtungsbedingungen abhängige, hohe Toxoidausbeute, verglichen mit dem Wildtypstamm E.coli 711/P307.

Die Prüfung der beiden Klone auf Toxizität des von ihnen synthetisierten Ent-LT Genprodukts erfolgte in einem in vitro Toxizitätstest an Y1-Zellen. Erwartungsgemäß zeigten nur pEFK700 tragende Keime die für Enterotoxineinwirkung charakteristischen morphologischen Veränderungen der adrenalen Y1-Zellen.

Die Herstellung eines weiteren Enterotoxoid-produzierenden Bakterien stammes mit hoher Toxoidsyntheserate gelang durch Ligierung des Ent-LT(B) Gens von pEFK295 mit dem Plasmidvektor pKS100.

DNA des Plasmids pEFK295 wurde mit der Restriktionsendonuklease HindIII verdaut und über eine Sephadex G100-Säule gereinigt. DNA des Plasmids pKS100 wurde ebenfalls mit HindIII verdaut und anschließend mit alkalischer Phosphatase aus Kälberdarm behandelt. Das pgal Plasmid pKS100 (konstruiert und erhalten von P. Starlinger; Trinks et al. [1981], Molec.Gen.Genet. 182, 183) ist ein Derivat des aus der Literatur bekannten multicopy Plasmids pBR322, aus dem der Bereich zwischen Nukleotid Nr. 4360 (EcoRI site) und Nukleotid Nr. 655 (HindII site) entfernt und durch ein EcoRI-HindII DNA-Fragment aus der DNA des Bakteriophagen lambda pgal 8 (Trinks et al. [1981], Molec.Gen.Genet. 182, 183) von ca. 4000 Nukleotiden Länge ersetzt wird. In den 4000 Nukleotiden ist das vollständige Galaktoseoperon des Bakteriums E.coli K12, einschließlich der Operator-Promoter Region, enthalten (Abb. 8). Die mit HindIII verdaute DNA von pEFK295 und die mit HindIII + Phospatase behandelte DNA von pKS100 wurden im Verhältnis 2:1 gemischt und mit T4 DNA Ligase religiert. Der E.coli Stamm F 165 Δ(gal) trp wurde mit dem Ligasegemisch transformiert und ampicillinresistente Zellen selektioniert.

Da pKS100 nur eine Erkennungsstelle für HindIII im GalE Gen enthält, können HindIII DNA-Fragmente aus pEFK295 nur in diese Stelle integrieren (Abb.8). Ampicillinresistente Klone von F165, die ein pKS100 Plasmid mit dem HindIII Fragment des Ent-LT-B Gens in der für die Transkription durch den Galaktose-Promotor richtigen Orientierung enthalten, wurden durch Spaltung der Plasmid-DNA mit verschiedenen Restriktionsendonukleasen und Auftrennung der DNA-Spaltstücke in Agarose- und Polyacrylamidgelen identifiziert.

Aus der Literatur (W.S. Dallas, S. Falkow [1980], Nature, 288, 499; E.K. Spicer et al. [1981], Proc. Natl. Acad. Sci. USA, 78, 50) ist bekannt, daß ein 780 bp langes HindIII-Fragment aus pEFK295 das Gen für das E.coli hitzelabile Enterotoxoid enthält, aber nur noch einen Teil des Gens für den toxischen Anteil des E.coli Entero-Holotoxins (Ent-LT-A). Das Genprodukt dieses HindIII-Fragments ist also atoxisch.

Ein Überproduzent für das E.coli Enterotoxoid wurde dadurch hergestellt, daß zusätzlich zum Gen für das Enterotoxoid (das 780 bp lange HindIII-Restriktionsfragment aus pEFK295) noch weitere drei HindIII-Fragmente aus dem pEFK295 Plasmid vor das Enterotoxoid-Gen eingesetzt wurden. Die Struktur des Plasmids aus enterotoxoidüberproduzierenden Keimen – mit der Bezeichnung pEBK620 – im Bereich der Integrationsstelle des Ent-LT-B-HindIII-Fragmentes wurde durch Spaltung der DNA mit HindII, HindIII, PstI, EcoRI, HaeIII (nicht eingezeichnet in Abb.9), HindII + HindIII, HindII + PstI, HindII + PstI + HaeIII, HindII + PstI + HindIII, AvaI + EcoRI sowie durch DNA-Sequenzanalyse ermittelt (Abb. 9 und 12). pEBK620 enthält neben dem HindIII-Fragment mit dem Ent-LT-B Gen noch drei weitere HindIII-Fragmente aus dem P307 Anteil von pEFK295: 2 x ein Fragment von 196 bp Länge in identischer Orientierung, das durch eine PstI-Stelle charakterisiert ist, und ein zweites Fragment von 111 bp Länge, dessen Lokalisation in P307 nicht bestimmt wurde (Abb. 9).

Aus dem Immunhämolyse-Testergebnissen ergibt sich folgendes Bild (siehe Tabelle 7).

TABELLE 7

| Keim | Ent-LT (Ü)[1] | Ent-LT (Z)[2] | % im Überstand |
|---|---|---|---|
| E.coli pEFK295 | 830 | 1360 | 40 |
| E.coli pEBK620 | 5000 | 12500 | 60 |

1) Enterotoxin-(Enterotoxoid)-Konzentration im Kulturüberstand nach Ab-

zentrifugation des Zellmaterials, angegeben in µg Choleratoxin-Äquivalenten pro Liter Kulturbrühe.

2) Intrazelluläre Enterotoxin-(Enteroxoid)-Menge aus zentrifugiertem, ultrabeschalltem Zellmaterial, angegeben in µg Choleratoxin-Äquivalenten pro Zellmasse, genommen aus 1 l Kulturbrühe.

Der extra- wie intrazelluläre Anteil an hitzelabilem Enterotoxoid liegt damit in dem durch Genrekombination neuhergestellten E.coli Keim um einen Faktor von etwa 10 höher als im toxinproduzierenden E.coli pEFK295.

Ein in vitro Toxizitätstest an adrenalen Y1-Zellen zeigte, daß Zellüberstände bzw. -lysate des Keimes mit dem Plasmid atoxisch sind. Die für Enterotoxineinwirkung charakteristischen morphologischen Veränderungen traten mit dem Toxoid des Keimes E.coli pEBK620 nicht auf.

Für die Herstellung einer Vakzine ist neben dem von den beschriebenen rekombinanten Plasmiden synthetisierten Enterotoxin bzw. -toxoid die Biosynthese eines Haftantigens erforderlich. Die Herstellung solcher Bakterien gelang im Fall der Plasmide pEFK500, pEFK600 und pEBK620 durch Transformation eines E.coli K12 5K Stammes, der bereits ein für ein schweinespezifisches K88-Antigen kodierendes Plasmid trug, mit den genannten Plasmiden. Im Falle des mit dem $P_L$-Promotor versehenen Enterotoxoidplasmids pEFK800 erfolgte diese Transformation erst, nachdem im Haftantigen-synthetisierenden Wirtsstamm das Repressorplasmid pRK248cIts ebenfalls durch Transformation etabliert worden war. Plasmid-DNA Präparationen und entsprechende Restriktionsanalysen zeigten, daß alle Plasmide innerhalb jeweils einer Bakterienzelle stabil waren. Diese Stabilität blieb auch nach einigen Passagen (mehr als 250 Generationszeiten) in antibiotikafreiem Medium erhalten.

Die Produktion des Enterotoxoids in Anwesenheit des entsprechenden K88-Plasmids wurde im Hämolysetest bestätigt. Auch die Synthese des K88-Antigens wurde durch die gleichzeitige Anwesenheit von pRK248clts bzw. der Enterotoxoidplasmide in der Zelle nicht beeinflußt.

In den nachfolgenden Beispielen, die die Erfindung näher erläutern, ihren Umfang aber in keiner Weise einschränken sollen, erfolgen alle Temperaturangaben in Celsiusgraden.

1.   Herstellung des Enterotoxin-produzierenden Bakterienstammes E. coli
     K12 5K/pEFK295

1.1  Isolierung und Charakterisierung des Enterotoxinplasmids P307 aus
     E. coli Wildtyp-Stämmen.

Der Enterotoxin-produzierende E. coli Stamm E. coli K12 711/P307 (tre⁻, phe⁻, pro⁻, his⁻, lac⁻, nal$^r$) stammt von Stanley Falkow, University of Washington, USA. Das Enterotoxinplasmid P307 wird zunächst aus dem schweinepathogenen Bakterienstamm E. coli 711 nach der Methode von Clewell und Helinski (Proc. Natl. Acad. Sci. USA $\underline{62}$/1159) isoliert:

4x1 ml der entsprechenden Keimsuspension werden in je 100 ml Trypticase Soy Broth Medium (TSB-Medium, Fa. Becton, Dickinson u. Co) aufgeschwemmt und 15 Stunden lang bei 37° im New Brunswick Inkubator unter Schütteln (120 upm) bebrütet. Nach Einbringen der 4 Vorkulturen in 4 x 1 l TSB-Medium erfolgt weitere Inkubation unter gleichen Bedingungen. Nach raschem Abkühlen der Kolben in Eiswasser werden die Keime innerhalb der logarithmischen Wachstumsphase durch 20 minütige Zentrifugation bei 13.700 upm und 4° geerntet. Bei einigen DNA-Präparationen findet die Ernte in der stationären Phase statt. Das so erhaltene Zellmaterial wird mit je 40 ml 50 mM Tris-HCl (pH = 8,0) gewaschen.

Der Zellaufschluß erfolgt in mehreren Schritten:

1.   Suspension von je 4 g Naßzellen in je 80 ml 25 % Saccharose/50 mM
     Tris-HCl, pH = 8,0.

2.   Zusatz von je 16 ml Lysozymlösung (10 mg in 2 ml, 250 mM Tris-HCl,
     pH = 8,0; Enzym von Serva, Heidelberg).

3.   Zusatz von je 32 ml 250 mM Na$_2$·EDTA, pH = 8,0.

4.   Zusatz von je 64 ml Detergenslösung: 1 % Brij 58 (Serva, Heidelberg),
     0,4 % Natriumdesoxycholat (Serva, Heidelberg), 62,5 mM Na$_2$·EDTA in
     50 mM Tris-HCl, pH = 8,0.

Zwischen den einzelnen Schritten wird jeweils eine Inkubationszeit von 5 Minuten bei 0° (Eisbad) eingehalten. Die Detergenszugabe erfolgt langsam und unter Rühren der Suspension.

Nach 25-minütiger Zentrifugation der stark viskosen Bakterienlysate bei 11.000 upm und 4° wird der Überstand abdekantiert, auf mehrere Zentrifugenröhrchen verteilt und mit je 2,5 ml einer gesättigten Cäsiumchloridlösung unterschichtet. Die anschließende, fünfzehnstündige Zentrifugation des geklärten Lysats im Beckman Ausschwingrotor SW27 erfolgt bei 50.000 upm und 4° in einer Beckman Ultrazentrifuge. Nach beendetem Lauf kann ein Großteil des Überstandes über eine Wasserstrahlpumpe abgesaugt werden, weil das gesamte DNA-Material im Cäsiumchloridpolster konzentriert ist. Die Abtrennung der ccc-Plasmid-DNA von der chromosomalen DNA erfolgt mit Hilfe von Cäsiumchlorid-Dichtegradientenzentrifugationen. Zu diesem Zweck werden die gesamten DNA-Konzentrate mit festem Cäsiumchlorid auf einen Brechungsindex von n = 1,391 eingestellt. Nach Zusatz von 2,5 ml einer wäßrigen Äthidiumbromidlösung (10 mg/ml, Serva, Heidelberg) pro 100 ml Gesamtvolumen erfolgt die Zentrifugation in einer Beckman Ultrazentrifuge bei 150.000 upm. Die Zentrifugationsdauer beträgt zwischen 50 und 60 Stunden bei 4°. Nach Beendigung der Gradientenzentrifugation lassen sich zwei verschiedene DNA-Banden unterscheiden. Die untere der beiden Banden enthält die gewünschte Plasmid DNA.

Die Gradienten werden fraktioniert und die DNA-hältigen Fraktionen bei 150.000 upm und 4° weitere 50 Stunden lang rezentrifugiert. Nach Isolierung der ccc-Plasmid-DNA Bande wird das Äthidiumbromid durch mehrmalige Extraktion mit Isopropanol, das vorher mit einer konzentrierten Cäsiumchloridlösung gesättigt worden ist, entfernt. In einer anschließenden Dialyse gegen 10 mM Tris-HCl, 0,1 mM $Na_2$.EDTA, pH = 8,0, wird das in der DNA-Lösung vorhandene Cäsiumchlorid beseitigt.

Die Bestimmung der DNA-Konzentrationen erfolgt durch Extinktionsmessung bei 260 nm im Zeiss PMQ 3 Spektralphotometer unter der Annahme, daß einem Extinktionswert von 20 eine DNA-Menge von 1 mg/ml entspricht.

Um die Plasmid-DNA auf chromosomale Verunreinigungen zu prüfen, wird eine Probe jeder DNA-Präparation im Agarosegel elektrophoretisch aufgetrennt. Die Elektrophoresen werden in vertikalen Plexiglasapparaturen mit einer effektiven Gellänge von 13 cm durchgeführt.

Präparationen der Gele (1 % Agarose): 3 g Agarose (Seakem, Rockland Maine, USA) werden nach Zusatz von 30 µl Äthidiumbromidlösung (10 mg/ml) in 300 ml "Loening-Puffer" gelöst und unter Rühren und Verwendung eines Rückflußkühlers 10 Minuten lang gekocht.

Loening-Puffer:          36 mM Tris-HCl, pH = 7,8
                         30 mM $NaH_2PO_4$
                         0,1 M $Na_2 \cdot EDTA$

Nach Ausgießen der unteren Pufferkammer bis zu einer Höhe von ungefähr 1 cm und dreißigminütiger Äquilibrierung der Agaroselösung bei 50° kann das Laufgel gegossen werden, nachdem der Gelsockel in der unteren Kammer erstarrt ist. In das noch flüssige Gel wird ein Plexiglaskamm (Weite der Probentaschen 7 mm) eingefügt. Nach dem Erstarren und vorsichtiger Entfernung des Gelkamms erfolgt das Auftragen der DNA-Proben durch Unterschichten unter vorher eingefüllten Elektrophoresepuffer. Die DNA-Proben bestehen aus:

A:    10 µl Plasmid-DNA (1,5 - 3,5 µg),
      + 40 µl Stopreagens
      Stopreagens:   50 % Saccharose
                     5 % N-Lauroylsarcosin (Sigma, St. Louis, USA)
                     0,5 % Bromphenolblau (Serva, Heidelberg)
                     in 0,1 M $Na_2 \cdot EDTA$

B:    10 µl chromosomale DNA (3,0 - 5,0 µg)
      + 40 µl Stopreagens.

Die Elektrophoresen werden 15 Stunden lang bei 2,3 V/cm und Raumtemperatur durchgeführt und die DNA-Banden anschließend auf einen UV-Durchleuchtungsschirm (Chromato-Vue C-62, San Gabriel, USA) sichtbar gemacht. Die auf die beschriebene Weise hergestellte P307-DNA erweist sich als frei von chromosomalen Verunreinigungen.

Die gereinigte Enterotoxinplasmid-DNA wird anschließend mit Hilfe der Restriktionsendonukleasen EcoR1 und BamHI und Auftrennung der einzelnen Fragmente im Agarosegel charakterisiert. Dabei werden jeweils 2 µg der gereinigten Plasmid-DNA in einem Gesamtvolumen von 50 µl (eingestellt mit dem entsprechenden Puffer) und pro µg DNA je eine Einheit des entsprechenden Enzyms eingesetzt. Die Inkubationszeit beträgt jeweils 1 Stunde bei 37°. Die Zusammensetzung der Puffergemische ist für die einzelnen Enzyme unterschiedlich und entspricht für das Restriktionsenzym EcoRI 100 mM Tris-HCl, pH = 7,5, 50 mM NaCl, 10 mM $MgCl_2$; für das Restriktionsenzym BamHI 6 mM Tris-HCl, pH = 7,5, 20 mM KCl, 6 mM $MgCl_2$ und 6 mM Dithioerythritol. Die Zugabe von 20 µl Stopreagens beendet die Spaltungsreaktionen.

Die nach Agarosegelelektrophorese in 1 % Agarose erhaltenen Spaltungsmuster stimmen mit den veröffentlichten Ergebnissen von Dallas et al. (J. Bact. 139/850) überein. Demnach befindet sich das Gen für hitzelabiles Enterotoxin zur Gänze auf einem BamHI Bruchstück mit einer relativen Molekülmasse von 5,8 Md des Plasmids P307.

1.2 Ligierung der Ent-LT Genregion mit dem Vektorplasmid pBR325 Das beschriebene DNA-Fragment mit einer relativen Molekülmasse von 5,8 Md wird anschließend mit dem in der Literatur beschriebenen Plasmidvektor pBR325 ligiert. pBR325 liegt in einer erhöhten Kopienanzahl von 25 - 30 pro Zelle vor. Dabei wird experimentell folgendermaßen vorgegangen:

3,5 µg pBR325-DNA bzw. 12,5 µg P307-DNA werden getrennt in Eppendorf-Röhrchen mit je 4 Einheiten der Restriktionsendonuklease BamHI in

einem Endvolumen von 50 µl entsprechend der beschriebenen Methode verdaut. Der Ansatz, welcher die Vektor-DNA enthält, wird nach beendeter Restriktionsverdauung weitere 30 Minuten bei 37° mit einer Einheit bakterieller alkalischer Phosphatase behandelt, um durch Abspaltung eines Phosphatrestes eine Religierung des Vektorplasmids im anschließenden Ligierungsexperiment zu verhindern. Beide Proben werden anschließend vereinigt, 40 µl TE-Puffer (10 mM Tris-HCl, pH = 7,5, 0,1 mM Na$_2$.EDTA) zugesetzt und anschließend sofort phenolisiert. Die Phenolextraktion erfolgt zweimal mit je 300 µl wassergesättigtem, frisch destilliertem Phenol. Nach zweimaliger Extraktion der wäßrigen Phase mit je 200 µl Diäthyläther und Zusatz von 1/10 des Volumens einer 2 M Natriumazetatlösung, eingestellt auf einen pH-Wert von 6,0, werden die DNA-Fragmente mit 3 Volumsanteilen von absolutem Äthanol gefällt. Die Fällung erfolgt 10 Minuten in einem Trockeneisbad, wobei die Proberöhrchen alle 2 Minuten geschwenkt werden, um ein Einfrieren zu verhindern. Nach einer 5-minütigen Zentrifugation in einer Eppendorf Zentrifuge wird der Überstand mit einer Pasteurpipette abgehoben und der Zentrifugationsrückstand mit 96 %igem Äthanol gewaschen. Nach erneuter Kühlung in Trockeneis und Zentrifugation wird der Überstand abgehoben und der Niederschlag im Exsiccator unter Vakuum 15 Minuten getrocknet.

Der getrocknete Rückstand wird anschließend in 60 µl frischem Ligasepuffer aufgenommen. Der Ligasepuffer hat folgende Zusammensetzung: 65 mM Tris-HCl, pH = 7,6, 7 mM MgCl$_2$, 10 mM Dithioerythritol in destilliertem, sterilisiertem Wasser. Die Ligierungsreaktion erfolgt 1 Stunde bei 22° nach Zusatz von 2 µl einer 10 mM ATP-Lösung und 2 µl T4-Ligase (BRL, Rockville, USA). Anschließend wird die Probe bei 4° über Nacht aufbewahrt.

1.3 Transformation von E. coli-Zellen mit der in vitro-rekombinierten DNA

E. coli K12 5K Zellen werden mit dem ligierten DNA-Gemisch transformiert. Um die Zellen in einen kompetenten Zustand überzuführen, werden die Keime aus 2 ml einer mittellogarithmischen Kultur zentrifugiert und in 10 ml einer sterilen, eisgekühlten 50 mM CaCl$_2$-Lösung suspendiert. Die Suspension wird anschließend bei 6.000 rpm und 4° 10 Minuten lang in einer Christ-Laborzentrifuge zentrifugiert, die Zellen erneut in $CaCl_2$-Lösung suspendiert und nochmals zentrifugiert. Das Zellpellet wird schließlich in 2 ml einer 50 mM $CaCl_2$-Lösung aufgenommen und im Eisbad mit 25 µl des gekühlten ligierten DNA-Gemisches und 3 µl einer 250 mM $Na_2$.EDTA-Lösung versetzt. Nach etwa einminütigem, raschem Erhitzen der Suspension auf 43° wird die Suspension mit 3 ml TSB-Medium versetzt und 3 Stunden bei 37°, ohne zu schütteln aber unter gelegentlichem Schwenken, bebrütet. Die Zellsuspension wird zentrifugiert, in 1 ml TSB-Medium aufgenommen, je 0,1 ml dieser Keimsuspension auf Selektionsplatten ausgestrichen und über Nacht bei 37° bebrütet. Als Selektionsplatten finden Blutagarplatten mit je 50 µg/ml Ampicillin Verwendung. Auf diesen Platten sind nur Transformanten zum Wachstum befähigt, d.h. Bakterien, die Plasmid-DNA aus der Lösung aufgenommen haben und deren Plasmide auch in den Keimen repliziert werden können.

Die auf den Selektionsplatten erhaltenen Transformanten werden anschließend mit sterilen Zahnstochern gleichzeitig auf ampicillinhältige Platten und auf solche, die 50 µg/ml Tetracyclin enthalten, überimpft. Nach Bebrütung dieser Agarplatten werden die tetracyclinsensitiven Kolonien in je 2 ml TSB-Medium über Nacht angezüchtet.

1.4. <u>Analyse der isolierten Plasmid-Rekombinanten</u>

Alle tetracyclinsensitiven Rekombinanten werden in einem analytischen Minilysatverfahren untersucht und ihre Plasmid-DNA in Agarosegelen charakterisiert. Dabei wird folgendermaßen vorgegangen:

Ca. 1 ml der angezüchteten Übernachtkultur wird in Eppendorf Röhrchen zentrifugiert, der Überstand mit einer Pasteurpipette abgesaugt und die Zellen in je 100 µl Lysozymlösung suspendiert. Die Lysozymlösung hat folgende Zusammensetzung: 20 mg Lysozym, 500 µl 1 M Glukose, 10 ml 0,1 M $Na_2$.EDTA, 250 µl 1 M Tris-HCl, pH = 8,0. Nach 30-minütigem Stehen im Eis erfolgt ein Zusatz von 200 µl einer Lösung, die durch Zusammenmischen von 2 ml einer 1 M Natriumhydroxidlösung, 2 ml einer 5 %igen SDS Lösung und 5,5 ml destillierten Wassers erhalten wird. Nach 5 Minuten weiteren Stehens im Eis werden 150 µl einer 3 M Natriumazetatlösung, pH = 4,8, zugesetzt. Das Gemisch wird weitere 60 Minuten im Eisbad belassen und anschließend 5 Minuten in einer Eppendorf Zentrifuge zentrifugiert. 400 µl des zentrifugierten Überstandes werden schließlich in ein neues Eppendorf Röhrchen überführt und die DNA mit 1 ml 96 %igem Äthanol bei -20° 30 Minuten lang gefällt. Nachdem zwei weitere Male auf die beschriebene Weise umgefällt wurde, wird das DNA-Pellet 15 Minuten im Vakuumexsiccator getrocknet, anschließend in 40 µl destilliertem Wasser gelöst und mit 2 µl pankreatischer RNase (1 mg/ml in 5 mM Tris-HCl, pH = 8,0) (Serva, Heidelberg) 30 Minuten lang bei 37° inkubiert.

Restriktionsverdauung mit der Restriktionsendonuklease BamHI und Agarosegelelektrophorese werden anschließend wie beschrieben durchgeführt. Zwei der auf diese Weise untersuchten Bakterienklone enthalten ein rekombiniertes Plasmid, welches ein BamHI Restriktionsfragment mit einer relativen Molekülmasse von 5,8 Md enthält. Aus diesen beiden Keimen wird die Plasmid-DNA durch Cäsiumchlorid-Dichtegradientenzentrifugation in Gegenwart von Äthidiumbromid wie beschrieben isoliert und neuerlich durch Spaltung mit verschiedenen Restriktionsendonukleasen charakterisiert. Dabei zeigt sich, daß die Plasmide dieser beiden Klone identisch sind und daß es sich bei den Plasmiden tatsächlich um DNA-Rekombinationen zwischen dem Vektorplasmid pBR325 und jenem DNA-Fragment des Enterotoxinplasmids P307 handelt, auf dem die Gene für hitzelabiles Enterotoxin liegen.

1.5 Test der durch in vitro-Genrekombination erzeugten Bakterienklone auf Enterotoxinproduktion im passiven Immunhämolysetest

Die beiden isolierten Bakterienklone werden anschließend auf die Produktion von hitzelabilem Enterotoxin geprüft. Als Test auf gebildetes Ent-LT findet ein passives Immunhämolyseverfahren Verwendung. Im Prinzip nützt dieser Test die Bindungsfähigkeit von hitzelabilem Enterotoxin an Schaferythrozyten und die immunologische Kreuzreaktivität von Enterotoxin mit gegen Choleratoxin gerichteten Antikörpern aus. Experimentell wird folgendermaßen vorgegangen:

Zunächst werden Blutagarosediffusionsplatten hergestellt. Dazu werden 250 ml frisches Hammelblut 3x mit dem gleichen Volumen PBS-Puffer gewaschen (8 g/l NaCl, 0,2 g/l $KH_2PO_4$, 0,2 g/l KCl und 1,15 g/l $Na_2HPO_4$, pH = 7,2). Nach Zentrifugation bei maximal 3.000 upm im GS 3 Rotor einer Sorvall Zentrifuge wird der Überstand entfernt und der Rückstand mit PBS-Puffer wieder auf 150 ml aufgefüllt. Eine 1 %ige Agaroselösung, der 20 mg/100 ml Natriumazid zugesetzt worden sind, wird auf 95° im Wasserbad erhitzt und anschließend bei Raumtemperatur auf 56° abgekühlt. Ein Teil des gewaschenen Bluts und 5 Teile dieser Agaroselösung werden bei 56° im Wasserbad vermischt und in 10 ml Portionen in Petrischalen abgefüllt. Mit Schablonen und Lochstanzern werden schließlich in die erstarrten Agaroseplatten Löcher gestanzt, um anschließend die Probelösung aufzunehmen.

Die Probelösungen werden folgendermaßen gewonnen: Jeweils 100 ml einer auf konstante Keimzahl (ca.$1 \times 10^8$/ml) eingestellten Bakterienkultur des zu untersuchenden Stammes werden steril filtriert, der Überstand lyophilisiert und die Menge des Lyophilisats bestimmt. Der Zellrückstand wird in 4 ml PBS-Puffer suspendiert und 3 x 20 Sekunden lang im Eisbad ultrabeschallt. Die geöffneten Bakterienzellen werden abzentrifugiert und der Überstand ebenfalls lyophilisiert, sowie die Menge des Lyophilisats bestimmt. Die Anzucht der auf Enterotoxin zu untersuchenden Keime erfolgt in einem Medium folgender Zusammensetzung (Finkelstein-Medium):

$Na_2HPO_4$ 5 g/l, $K_2HPO_4$.$3 H_2O$ 6,55 g/l, $NH_4Cl$ 1,18 g/l, $Na_2SO_4$ 0,089 g/l, $MgCl_2$ 0,042 g/l, $MnCl_2$ 0,004 g/l, $FeCl_3$ 0,005 g/l, Casamino acids (Difco) 10,0 g/l, Thiaminiumhydrochlorid (Merck, BRD) 1 mg/l, Glukose 1,0 g/l.

Die Probelyophilisate von Überständen und Zellysaten werden in einer Konzentration von 200 mg Lyophilisat pro ml PBS-Puffer aufgelöst und 3 x jeweils 1:1 mit PBS-Puffer verdünnt, so daß eine Verdünnungsreihe aus 4 Einzelproben hergestellt wird. Jeweils 10 µl der einzelnen Verdünnungen werden in jeweils eine aus der Blutagaroseplatte ausgestanzte Öffnung aufgetragen und für 15-20 Stunden bei 37° inkubiert. Nach beendeter Diffusion der Probelösung werden die Blutagaroseplatten mit folgender Lösung überschichtet: 0,5 % Agarose + 20 mg/100 ml Natriumazid werden aufgeschmolzen, auf 56° abgekühlt und in 3,3 ml Portionen auf 56° im Wasserbad belassen. Je 150 µl Meerschweinchenkomplement (Gibco, USA) sowie 100 µl eines gegen Vibrio-Choleratoxin gerichteten Ziegenserums werden den einzelnen Portionen zugesetzt und auf die vorbehandelten Blutagaroseplatten verteilt. Nach Erstarren der oberen Schicht wird eine weitere Stunde bei 37° bebrütet. Enterotoxinhältige Proben führen zur Ausbildung von Hämolysehöfen, deren Durchmesser der Konzentration an Enterotoxin proportional sind. Im Vergleich mit einer Standardverdünnungsreihe, die durch Verdünnen einer Lösung von Vibrio-Choleratoxin (Schwarz-Mann) bekannter Konzentrationen erhalten wird, ist eine quantitative Auswertung der Versuche möglich. Schon nach den ersten Hämolysetests zeigt sich, daß in den Lysaten ultrabeschallter Zellen der beiden Klone eine wesentlich höhere Menge an Enterotoxin nachgewiesen werden kann, als dies in entsprechenden Zellysaten aller untersuchten Ent-LT Wildtyp-Stämmen der Fall ist. Ein quantitativer Vergleich der synthetisierten Enterotoxinmenge in den Lyophilisaten der entsprechenden Zellüberstände ist zunächst nicht möglich, weil die Enterotoxinkonzentration in den Zellüberständen der Wildtyp-Kulturen unter der Nachweisgrenze des beschriebenen Tests liegt. Aus vergleichenden Schätzungen der intrazellulären Enterotoxinanteile kann der Schluß gewonnen werden, daß die Menge an hitzelabilem Enterotoxin in den durch Genrekombination hergestellten E. coli Keimen um einen Faktor von etwa 50 höher ist als im entsprechenden Wildtyp-Stamm E. coli 711/P307.

Aus den Ergebnissen der beschriebenen Restriktionsanalysen kann geschlossen werden, daß das als pEFK295 bezeichnete in vitro rekombinierte Hybridplasmid das gesamte Strukturgen für hitzelabiles Enterotoxin trägt. Außerdem ist auch die Orientierung des klonierten BamHI-Fragments durch Restriktionsanalysen eindeutig festgelegt. Aus den erhaltenen Analyseergebnissen sowie den von Dallas et al.(J.Bact.139/850) publizierten Daten kann eine physikalische Genkarte von pEFK295 erstellt werden.

2.    Herstellung des Enterotoxoid-produzierenden Bakterienstammes E.
      coli K12 5K/pEFK500


2.1.  Gezielte Entfernung des für die Enteropathogenität verantwortlichen
      Ent-LT Genanteils aus dem Plasmid pEFK295

Der gewählte experimentelle Weg besteht in der enzymatischen Öffnung des Plasmids pEFK295 mit der Restriktionsendonuklease EcoRI und anschließendem Ringschluß des größeren der dabei entstehenden Fragmente durch Ligierung mit T4-Ligase. Um zu gewährleisten, daß eine solche Deletion mit erhöhter Wahrscheinlichkeit stattfindet, werden 2,5 µg gereinigter pEFK295-DNA mit EcoRI vollständig gespalten und anschließend in einem Volumen von 150 µl ligiert. Sämtliche anderen experimentellen Bedingungen für Restriktionsverdauung, Phenolextraktion, DNA-Fällung und Ligierung bleiben unverändert und entsprechen der zur Herstellung des Hybridplasmids pEFK295 beschriebenen Vorgangsweise. Nach Transformation kompetenter E. coli K12 5K Zellen mit der im Ligasegemisch anwesenden DNA werden auf die beschriebene Weise alle jene Transformanten selektioniert, welche Ampicillinresistenz aufweisen, aber die Chloramphenicolresistenz verloren haben. Aus diesen Keimen wird die Plasmid-DNA isoliert und die beabsichtigte Deletion im neu entstandenen Plasmid mit der Bezeichnung pEFK500 durch vergleichende Spaltung der Plasmide pEFK295 und pEFK500 mit den Restriktionsendonukleasen EcoRI, BamHI und HindIII eindeutig bestätigt. Es liegt somit ein durch in vitro Genrekombination künstlich hergestelltes Plasmid vor, welches die genetische Information für die bindende Proteinuntereinheit des hitzelabilen E. coli Enterotoxins besitzt. Jene DNA-Region, welche für die A-Untereinheit des Enterotoxins, die für die Toxizität verantwortlich ist, kodiert, ist mit Hilfe des beschriebenen Deletionsexperiments aus dem Plasmid pEFK295 entfernt worden.


Eine korrekte Transkription des verbleibenden Enterotoxingenfragments ist allerdings nur von einem geeigneten Promotor des Vektorplasmids, mögli-

cherweise dem Chloramphenicol-Acetyltransferase-Promotor, zu erwarten, wobei vorauszusetzen ist, daß der korrekte Leserahmen ebenfalls erhalten geblieben ist. Tatsächlich ist ein im Anschluß an das Klonierungsexperiment durchgeführter passiver Immunhämolysetest für pEFK500-tragende E. coli Keime positiv. Die Ausbeuten sind mit jenen der pEFK295-tragenden Keime vergleichbar.

## 2.2 Biologische Tests der durch in vitro-Genrekombination hergestellten Bakterienklone

Um zu bestätigen, daß es sich bei pEFK500-tragenden Keimen tatsächlich um Bakterienklone handelt, die imstande sind, ein atoxisches aber immunologisch aktives Enterotoxoid zu synthetisieren, wird ein biologischer Enterotoxintest in Jejunum-Ligaturen von Kaninchen durchgeführt. Neben Choleratoxin als positiver und plasmidfreien E. coli Keimen als negativer Kontrolle wird der Wildtyp Enterotoxinproduzentenstamm E. coli 711/P307 neben den durch in vitro-Genrekombination erhaltenen Stämmen E. coli K12 5K/pEFK295 und E. coli K12 5K/pEFK500 eingesetzt.

Der Test an ligierten Dünndarmschlingen von Kaninchen wird folgendermaßen durchgeführt:

Für jeden Test werden jeweils 6 Chinchillakaninchen, ca. 1,7 - 2,2 kg schwer, 48 Stunden lang ohne Nahrungsaufnahme belassen, wobei Wasser weiter ad libidum angeboten wird. Kurz vor der Operation wird die gesamte Bauchregion des Kaninchens rasiert. Die Operation erfolgt unter Inhalationsnarkose mit Halothan (ungefähr 3,5 Volumsprozent). Nach je einem Hautanstrich mit Incidin und Merfen-Orange und einem Hautschnitt in der Medianlinie wird die Bauchhöhle entlang der Linea alba ca. 6 - 7 cm weit eröffnet und das gesamte Jejunum vorverlagert. Das Jejunum wird cranial etwa in der Höhe des ersten Peyer'schen Plaque's ligiert und von cranial nach caudal etwa in seiner halben Länge mit 20 ml steriler 0,86 %iger NaCl-Lösung gespült. Der gespülte Darmabschnitt wird caudal mit einer Doppelligatur abgebunden und von dort ab in cranialer Richtung 6 x im Abstand von ca. 8 - 10 cm weitere Doppelligaturen gesetzt. Für sämtliche Darmligaturen wird Catgut Nr. 0 verwendet. In den ligierten Darmschlin-

gen 1 - 6 werden 5 x je 1 ml der Testsubstanz und 1 x 1 ml PBS-Puffer injiziert. Die Reihenfolge der Inokulation wird bei jedem Kaninchen geändert, so daß schließlich jede Substanz einmal in die Darmschlingen 1, 2, 3, 4, 5 und 6 appliziert wird. Anschließend wird der Dünndarm wieder in die Bauchhöhle zurückverlagert und die Bauchdecke mit einer Peritonaeum-Muskelnaht und einer Hautnaht (beides einfache Kopfnähte mit Seide Nr. 2) verschlossen. Nach ca. 18 Stunden werden die Kaninchen mit T-61 (i. cardial) getötet, das Jejunum entnommen und die Länge der abgebundenen Darmschlingen sowie die darin enthaltene Flüssigkeitsmenge gemessen. Daraus wird der Quotient

$$\frac{\text{Darmschlingeninhalt (ml)}}{\text{Darmschlingenlänge (cm)}}$$

errechnet. Dieser Quotient stellt ein direktes Maß für die Fähigkeit der applizierten Substanz dar, das Einströmen von Flüssigkeit in das Darmlumen zu induzieren (Enterotoxizität). Die Auswertung dieses Tests mit Zellüberständen bzw. Zellyophilisaten der beschriebenen Keime ergibt eindeutig, daß sich sowohl Zellüberstände wie auch Lysate von Keimen mit den Plasmiden P307 sowie pEFK295 als toxisch erweisen, während Extrakte von pEFK500-tragenden Keimen atoxisch sind.

Diese Ergebnisse können in einem in vitro Toxizitätstest an Y1-Zellen bestätigt werden. Dieser Test beruht auf der morphologischen Veränderung von adrenalen Y1-Zellen infolge des durch Enterotoxin bewirkten intrazellulären Anstiegs der cAMP-Konzentration.

Der Test wird folgendermaßen durchgeführt:
100 ml einer Übernachtkultur des jeweiligen Testkeims in Finkelstein-Medium werden nach Eintritt in die stationäre Phase in einem Sorvall GSA-Rotor bei 9.000 upm zentrifugiert. Der Überstand wird anschließend über Nalgene 45 µ Sterilfilter filtriert. Der sterile Kulturüberstand wird lyophilisiert und das Gewicht des Lyophilisats bestimmt. Die Zellrückstände werden in je 4 ml PBS-Puffer aufgenommen und 60 Sekunden im Eis ultrabeschallt. Die erhaltenen Zellysate werden zentrifugiert, ebenfalls lyophilisiert und das Gewicht des Lyophilisats bestimmt. Der eigentliche Test wird in HAMS F-10 Medium mit 3 % inaktivem, fötalem Kälberserum

und Glutamin (Fa. GIBCO, Katalog-Nr. 430/1200) unter Zusatz von 1,2 g/l Natriumhydrogencarbonat durchgeführt. Vor der Verwendung im Test werden pro 100 ml dieses Mediums 1,5 ml einer Penicillin-Streptomycin-Lösung zugesetzt (100 Millionen Einheiten pro Liter Natriumpenicillin, 10 g/l Streptomycinsulfat). Der Test wird mit Y1-Zellen, die in Kunststoff-Mikrotiterplatten zu je 96 Positionen gezogen werden, ausgeführt. Zunächst wird die Flüssigkeit aus den einzelnen Probeöffnungen entfernt und sodann je 200 µl des beschriebenen HAMS-Mediums pro Öffnung einpipettiert. Von den zu untersuchenden Proben werden Verdünnungsreihen von jeweils 1:10 hergestellt, so daß die Proben in Konzentrationen von 0,1 µg/ml bis 10 µg/ml vorliegen. Je 10 µl der einzelnen Verdünnungen werden in die Mikrotiterplatten einpipettiert. Anschließend werden die Platten 6 Stunden lang bei 37° inkubiert und die Zellen sodann unter dem Mikroskop betrachtet. Die Anzahl der abgerundeten Y1-Zellen ist ein Maß für die Enterotoxinkonzentration in der Probe.

Die Auswertung des Y1-Tests ergibt, daß nur P307 bzw. pEFK295 tragende Keime die für Enterotoxineinwirkung charakteristischen morphologischen Veränderungen ergeben. Die mit E. coli K12 5K/pEFK500 behandelten Y1-Zellen zeigen keine morphologischen Veränderungen. Es besteht daher Übereinstimmung mit den Ergebnissen des biologischen Toxizitätstests an Kaninchen.

2.3. Abhängigkeit der Enterotoxin (bzw. -toxoid) Produktion von den Wachstumsbedingungen der Keime

Der Enterotoxingehalt aller Keime ist vom Nährmedium abhängig. Durch Zusatz von Ampicillin zu pEFK295 bzw. pEFK500 tragenden E. coli K12 5K Bakterienkulturen kann eine zusätzliche Steigerung der Toxinausbeuten erreicht werden.

| µg VCT/l | P307 | pEFK295 | pEFK500 |
|---|---|---|---|
| F+AB[1] | -- | 1137±40% | 1082±13% |
| F[2] | 24±38% | 628±28% | 353±60% |
| TSB+AB[3] | -- | 386±10% | 113±9% |
| TSB[4] | 0 | 47±26% | 0 |
| M9+AB[5] | -- | 1640±17% | 24±21% |
| M9[6] | 23±10% | 1644±10% | 50±10% |

1)   Finkelstein-Medium + 50 µg/ml Ap

2)   Finkelstein-Medium ohne Ap

3)   Trypticase Soy Broth + 50 µg/ml Ap

4)   Trypticase Soy Broth ohne Ap

5)   Synthetisches M9-Medium mit Glycerin als einziger C-Quelle + 50 µg/ml Ap

6)   Synthetisches M9-Medium mit Glycerin als einziger C-Quelle ohne Ap.


## 2.4.   Transformation von Haftantigen-synthetisierenden Bakterien mit dem Enterotoxoid-Plasmid pEFK500

Für eine Vakzine ist neben dem von pEFK295 oder pEFK500 kodierten Toxin bzw. Toxoid die Biosynthese eines Haftantigens erforderlich. Die Herstellung solcher Bakterien gelingt durch Transformation von E. coli K12 5K Zellen, die bereits für ein schweinespezifisches K88-Antigen kodierende Plasmide tragen, mit den Plasmiden pEFK295 bzw. pEFK500. E.coli K12 Stämme, die porcines Haftantigen der Serotypen K88 AB, K88 AC und K88 AD produzieren, stammen von P.Guinee, National Institute of Health, Bilthoven, Niederlande. Die Transformation dieser Stämme mit je 1 µg des Plasmids pEFK295 bzw. pEFK500 erfolgt auf die unter 1.3 beschriebene Weise. Nach erfolgter Transformation von K88 porcinem Haftantigen der Serotypen K88 AB bzw. K88 AC bzw. K88 AD synthetisierenden Keimen mit pEFK295 bzw. pEFK500 durchgeführte Plasmid-DNA Präparationen und entsprechende Restriktionsanalysen zeigen, daß beide Plasmide innerhalb jeweils einer Bakterienzelle stabil sind. Diese Stabilität bleibt auch nach einigen Passagen (mehr als 250 Generationszeiten) in antibiotikafreiem Medium erhalten.

Die Produktion des Enterotoxins bzw. Enterotoxoids in Anwesenheit des entsprechenden K88-Plasmids wird im passiven Hämolysetest, der wie unter 1.5 beschrieben durchgeführt wird, bestätigt. Auch die Synthese des K88-Antigens wird durch die gleichzeitige Anwesenheit von pEFK295 oder von pEFK500 in der Zelle nicht beeinflußt, wie immunelektrophoretisch gezeigt werden kann.

Der Elektroimmunoassay zur Haftantigenbestimmung wird folgendermaßen durchgeführt:

a) Reinigung des Antigens

K88-Antigen, das in relativ großer Menge in den Überständen der Fermentation des Stammes E.coli 0:8 K87,88ab H:19 vorliegt, wird in einem zweistufigen Trennverfahren, das einen Ultrazentrifugationsschritt und eine Adsorption an eine Immunosorbentsäule beinhaltet, gewonnen. Als Immunosorbent wird vorhandenes K12 K88 Serum, das mit K12 absorbiert war, verwendet.

Ultrazentrifugation: 1 g Trockensubstanz des vorhandenen Lyophilisates aus Überständen von E.coli P263 wird in 25 ml PBS def. gelöst und 30 Minuten bei Zimmertemperatur gerührt. Diese leicht trübe opaleszierende Lösung wird im Rotor 60 Ti 90 Minuten bei 45000 upm zentrifugiert und die klare überstehende Lösung für die weitere Reinigung verwendet.

Gewinnung von IgG aus Serum: 20 ml des mit K12 absorbierten K12 K88 Serums werden 48 Stunden gegen Phosphatpuffer (0,0175 M pH 6,3) dialysiert, zentrifugiert und anschließend auf eine mit Puffer äquilibrierte DEAE Sephacelsäule gebracht. IgG wird mit dem Ausschlußvolumen eluiert und besitzt einen hohen Reinheitsgrad (2 Banden im SDS Gel).

Kupplung von Immungiobulin G an aktivierte Sepharose: 15 g CNBr-akti-vierte Sepharose 4B (Pharmacia) werden mit 2 Liter $10^{-3}$ M HCl gequollen und gewaschen. Die lyophilisierte IgG-Präparation (190 mg Protein) wird in 50 ml $NaHCO_3$ (0,1 M $NaHCO_3$ + 0,5 M NaCl) gelöst, mit der gewaschenen aktivierten Sepharose gemischt und 18 Stunden bei 4° unter leichtem Rühren gehalten. Nach dem Absättigen der restlichen aktiven Gruppen mit Äthanolamin wird das Gel in eine Glassäule (Durchmesser 5 cm) gebracht, je dreimal mit Natriumazetatpuffer (pH 4) und Natriumboratpuffer (pH 8) gewaschen, einmal mit Kaliumthiocyanat (3 M) gewaschen und anschließend mit dem Laufpuffer (0,1 M Na-Phosphat, 0,5 M NaCl, pH 7,3) äquilibriert.

Durchführung des Absorptionsschrittes: Die zentrifugierte Probe (6 ml entspricht 250 mg Trockensubstanz) wird mit dem Laufpuffer auf 15 ml aufgefüllt und auf die Säule gepumpt. Nach dem Durchgang des Proteinpeaks wird mit 150 ml Puffer gewaschen und anschließend mit 150 ml 3 M KSCN eluiert. Die gegen Anti-K88 positiven Fraktionen werden ankonzentriert, mit der zehnfachen Menge Natriumphosphat, das 0,01 % ß-Mer-captoäthanol enthält, aufgefüllt und schließlich auf ein Endvolumen von 2,5 ml gebracht.

b) Charakterisierung des Antigens:

SDS Polyacrylamidgele: Im 10 %igen Acrylamidgel erhält man nach der Inkubation mit 0,1 % SDS und 0.1 % ß-Mercaptoäthanol eine Proteinbande, der ein Molekulargewicht von 23.000 d zuzuordnen ist.

Polyacrylamidelektrophorese in 8 M Harnstoff: Im 5 %igen Gel erhält man in Gegenwart von 8 M Harnstoff eine Proteinbande mit einem Rf-Wert von 0,25, bezogen auf die Wanderungsweite des Farbmarkers (Bromphenolblau).

Polyacrylamidelektrophorese im Nativgel:

Die im Nativzustand vorhandenen Aggregate sind so groß, daß ein Einwandern in ein 5 %iges Gel nicht möglich ist.

c) Elektroimmunoassay:

Prinzip: Die Probe wird auf der Anti-K88 hältigen Agarose aufgetragen und wandert im elektrischen Feld zur Anode. Das Antigen bildet dabei mit dem in der Agarose vorhandenen Antikörper Ag-Ab-Komplexe, die als stationäre Aggregate sichtbar werden. Die Höhe der Präzipitate mit der charakteristischen "Rocket" Form ist proportional der aufgetragenen Antigenmenge.

Methode: 1 g Agarose (Pharmacia Type C) wird in 100 ml Tris-Barbitalpuffer (pH 8,8) suspendiert, bei 90-95° im Wasserbad unter ständigem Rühren geschmolzen und anschließend auf 55° abgekühlt. 12 ml dieser geschmolzenen Agarose werden nach dem Zusatz des Antiserums (100 µl) in einer Eprouvette durch vorsichtiges Schwenken gemischt und auf eine Glasplatte (83 x 93 x 1 mm) gegossen. Nach dem Erkalten werden die Applikationslöcher mit einem Durchmesser von 2,5 mm gestanzt. Die zu analysierenden Proben müssen als klare Lösungen vorliegen (ev. Zentrifugation) und werden mit 10 M Harnstoff 1+1 gemischt und 30 Minuten bei 37° inkubiert. Verdünnungen werden in Tris-Barbital-Harnstoff-Puffer hergestellt. Je 5 µl der Proben werden mit einer Mikropipette aufgetragen, wobei die Verweilzeit bis zum Anlegen der Spannung nicht mehr als 5 Minuten betragen soll. Die Elektrophoresezeit beträgt bei 2 Volt/cm 16 Stunden, bei 10 Volt/cm 4 Stunden. Nach Beendigung des Laufens werden die Platten dreimal 10 Minuten mit Filterpapier gepreßt, einmal 20 Minuten mit NaCl 0,8 %ig, einmal 20 Minuten mit Wasser gewaschen, getrocknet und gefärbt. Die Auswertung erfolgt durch Ausmessen der durch die Präzipitationslinie umgrenzten Fläche, kann aber auch bei gleichmäßiger Rocketform durch Abmessen der Höhe erfolgen. Durch den Vergleich der unbekannten Probe mit einem bekannten Standardpräparat kann der Gehalt an K88-Antigen relativ genau bestimmt werden.

3. Herstellung des Enterotoxoid-produzierenden Bakterienstammes E.coli K12 5K/pEFK600

3.1 Spaltung des Ent-LT Plasmids pEFK295 mit den Restriktionsenzymen HindIII und Ligierung der DNA-Fragmente mit dem Vektorplasmid pBR322

3,5 µg pBR322-DNA bzw. 12,5 µg pEFK295-DNA werden getrennt in Eppendorf-Röhrchen mit je 4 Einheiten Restriktionsendonuklease HindIII in einem Endvolumen von 50 µl des von der Herstellerfirma angegebenen Puffers entsprechend der beschriebenen Methode verdaut. Der Ansatz, welcher die Vektor-DNA enthält, wird nach beendeter Restriktionsverdauung weitere 30 Minuten bei 37° mit einer Einheit bakterieller alkalischer Phosphatase behandelt, um durch Abspaltung eines Phosphatrestes eine Religierung des Vektorplasmids im anschließenden Ligierungsexperiment zu verhindern. Beide Proben werden anschließend vereinigt, 40 µl TE-Puffer (10 mM Tris-HCl, pH = 7,5, 0,1 mM $Na_2$.EDTA) zugesetzt und anschließend sofort phenolisiert. Die Phenolextraktion erfolgt zweimal mit je 300 µl wassergesättigtem, frisch destilliertem Phenol. Nach zweimaliger Extraktion der wäßrigen Phase mit je 200 µl Diäthyläther und Zusatz von 1/10 des Volumens einer 2 M Natriumazetatlösung, eingestellt auf einen pH-Wert von 6,0, werden die DNA-Fragmente mit 3 Volumsanteilen von absolutem Äthanol gefällt. Die Fällung erfolgt 10 Minuten in einem Trockeneisbad, wobei die Proberöhrchen alle 2 Minuten geschwenkt werden, um ein Einfrieren zu verhindern. Nach einer 5-minütigen Zentrifugation in einer Eppendorf Zentrifuge wird der Überstand mit einer Pasteurpipette abgehoben und der Zentrifugationsrückstand mit 96 %igem Äthanol gewaschen. Nach erneuter Kühlung in Trockeneis und Zentrifugation wird der Überstand abgehoben und der Niederschlag im Exsiccator unter Vakuum 15 Minuten getrocknet.

Der getrocknete Rückstand wird anschließend in 60 µl frischem Ligasepuffer aufgenommen. Der Ligasepuffer hat folgende Zusammensetzung: 65 mM Tris-HCl, pH = 7,6, 7 mM $MgCl_2$, 10 mM Dithioerythritol in destilliertem, sterilisiertem Wasser. Die Ligierungsreaktion erfolgt 1 Stunde bei 22° nach Zusatz von 2 μl einer 10 mM ATP-Lösung und 2 μl T4-Ligase (BRL, Rockville, USA). Anschließend wird die Probe bei 4° über Nacht aufbewahrt.

3.2 Transformation von E. coli-Zellen mit der in vitro-rekombinierten DNA

E. coli K12 5K Zellen werden mit dem ligierten DNA-Gemisch analog wie unter 1.3 beschrieben transformiert. Als Selektionsplatten finden Blutagarplatten mit je 50 μg/ml Ampicillin Verwendung. Die auf den Selektionsplatten erhaltenen Transformanten werden anschließend mit sterilen Zahnstochern gleichzeitig auf ampicillinhältige Platten und auf solche, die 50 μg/ml Tetracyclin enthalten, überimpft. Nach Bebrütung dieser Agarplatten werden die tetracyclinsensitiven Kolonien in je 2 ml TSB-Medium über Nacht angezüchtet.

3.3 Analyse der isolierten Plasmid-Rekombinanten

Alle tetracyclinsensitiven Rekombinanten werden auf Blutagarplatten mit je 30 μg/ml Chloramphenicol auf Chloramphenicolresistenz geprüft. Die Keime mit Chloramphenicol- und Ampicillinresistenz sowie ausschließlich ampicillinresistente Klone werden im analytischen Minilysatverfahren untersucht und ihre Plasmid-DNA in Agarosegelen charakterisiert.

Restriktionsverdauung mit der Restriktionsendonuklease HindIII und Agarosegelelektrophorese werden wie beschrieben, durchgeführt. Es werden 3 verschiedene Bakterienklone identifiziert, die Plasmide mit der Bezeichnung pEFK502 bzw. pEFK600 bzw. pEFK610 beherbergen (Abb. 4).

pEFK502 trägt jenen Teil des Ent-LT Gens (a), der für die toxische A-Untereinheit des hitzelabilen E.coli Eterotoxins kodiert, während in pEFK610 das Gen für die bindende B-Untereinheit integriert ist (b). Bei beiden Plasmiden stammt der Vektoranteil von pBR322. Das rekombinante Plasmid pEFK600 enthält ebenfalls nur mehr jenes HindIII-Fragment des Ent-LT-Plasmids pEFK295, welches das Gen für die B-Untereinheit trägt. Der Vektoranteil stammt in diesem Fall jedoch gleichfalls aus dem Plasmid pEFK295 und damit vom ursprünglich eingesetzten Plasmidvektor pBR325.

3.4 Test der durch in vitro-Genrekombination erzeugten Bakterienklone auf Enterotoxinproduktion im passiven Immunhämolysetest

Die isolierten Bakterienklone werden anschließend auf die Produktion von hitzelabilem Enterotoxin geprüft. Als Test auf gebildetes Ent-LT findet das beschriebene passive Immunhämolyseverfahren Verwendung. Die Klone mit den Plasmiden pEFK502 bzw. pEFK610 erweisen sich im Immunhämolysetest als negativ. Der Bakterienklon, welcher das Plasmid pEFK600 beherbergt, zeigt positive Hämolysereaktion. Die Enterotoxinausbeuten pEFK600 tragender Keime sind mit jenen der pEFK295 tragenden Keime vergleichbar.

3.5 Toxizitätstest des durch in vitro-Genrekombination hergestellten Bakterienklons

Um zu bestätigen, daß es sich bei pEFK600 tragenden Keimen um einen Bakterienklon handelt, der imstande ist, ein atoxisches, aber immunologisch aktives Enterotoxoid zu synthetisieren, wird ein in vitro-Toxizitätstest an Y1-Zellen durchgeführt. Neben Choleratoxin als positiver und plasmidfreien E.coli Keimen als negativer Kontrolle wird der Wildtyp-Enterotoxinproduzentenstamm E.coli 711/P307 neben den durch in vitro-Genrekomination erhaltenen Stämmen E.coli K12 5K/pEFK295 und E.coli K12 5K/pEFK600 eingesetzt. Der Test wird wie beschrieben durchgeführt. Die Auswertung des Y1-Tests ergibt, daß nur P307 bzw. pEFK295 tragende Keime die für Enterotoxineinwirkung charakteristischen morphologischen Veränderungen ergeben. Die mit E.coli K12 5K/pEFK600 behandelten Y1-Zellen zeigen keine morphologischen Veränderungen.

3.6. Transformation von Haftantigen-synthetisierenden Bakterien mit dem Enterotoxoid-Plasmid pEFK600

Für eine Vakzine ist neben dem von pEFK600 kodierten Toxoid die Biosynthese eines Haftantigens erforderlich. Die Herstellung solcher Bakterien gelingt durch Transformation von E.coli K12 5K Zellen, die bereits für ein schweinespezifisches K88-Antigen kodierende Plasmide tragen, mit dem Plasmid pEFK600. E.coli K12 Stämme, die porcines Haftantigen des Serotyps K88(AB) produzieren, stammen von P. Guinee, National Institute of Health, Bilthoven, Niederlande. Die Transformation dieser Stämme mit je 1 μg des Plasmids pEFK600 erfolgt auf die beschriebene Weise. Eine nach erfolgter Transformation von K88 porcinem Haftantigen des Serotyps K88(AB) synthetisierenden Keimen mit pEFK600 durchgeführte Plasmid-DNA-Präparation und entsprechende Restriktionsanalysen zeigen, daß beide Plasmide innerhalb jeweils einer Bakterienzelle stabil sind. Diese Stabilität bleibt auch nach einigen Passagen (mehr als 250 Generationszeiten) in antibiotikafreiem Medium erhalten.

Die Produktion des Enterotoxoids in Anwesenheit des entsprechenden K88-Plasmids wird im passiven Hämolysetest bestätigt. Auch die Synthese des K88-Antigens wird durch die gleichzeitige Anwesenheit von pEFK600 in der Zelle nicht beeinflußt, wie im Hämagglutinationstest gezeigt werden kann. Haftantigene vom K88-Typ führen zu einer spezifischen mannoseresistenten Hämagglutination von Hühnererythrozyten. Der Test wird folgendermaßen durchgeführt:
Zunächst werden die zu untersuchenden Keime auf speziellen Agarplatten ausgestrichen. Das Agarmedium hat folgende Zusammensetzung:
1 % Casamino acids, 0,15 % Hefeextrakt, 0,005 % $MgSO_4$, 0,0005 % $MnCl_2$, 2 % Agar (Difco).
Die betreffenden Platten werden 18 Stunden lang bei 37° bebrütet.

Herstellung der Hühnererythrozyten:
9 Teile frisch genommenes Hühnerblut werden mit einem Teil 3,8 %iger Natriumcitratlösung versetzt. Die Suspension wird 10 Minuten lang bei 6.000 upm in einer Laborzentrifuge bei 4° zentrifugiert. Der klare, leicht gelbliche Überstand wird abgesaugt und die gepackten Erythrozyten 2 x mit physiologischer Kochsalzlösung gewaschen. Die Erythrozytensuspension

wird mit physiologischer Kochsalzlösung auf das ursprüngliche Volumen aufgefüllt und weitere 2 x wie beschrieben zentrifugiert und gewaschen. Nach der letzten Zentrifugation wird mit physiologischer Kochsalzlösung eine 3-volumsprozentige Erythrozytensuspension hergestellt. Die Erythrozytensuspension wird in 2 gleiche Teile geteilt und zu einem Teil Mannose in einer Endkonzentration von 0,5 % zugesetzt.

In vorgekühlte Kunststoffmikrotiterplatten zu je 96 Positionen mit konisch zulaufendem Boden werden pro Probenöffnung je 50 µl einer physiologischen Kochsalzlösung einpipettiert, wobei die Lösung in jeder zweiten Position 5 %ig an Mannose ist. Sodann werden in jede Probenöffnung weitere 50 µl der zu untersuchenden Keimsuspension einpipettiert. Die Keimsuspension wird dadurch hergestellt, daß je eine Öse des zu testenden Keimes von den Agarplatten abgenommen und in je 1 ml physiologischer Kochsalzlösung suspendiert wird. Die Mikrotiterplatten werden bei 4° ca. 1/2 Stunde inkubiert. Anschließend werden die Mikrotiterplatten in einer flachen Schale auf Eis gelegt und je 50 µl der vorgekühlten Erythrozytenlösung zugesetzt. Nach kurzem Aufschütteln wird weitere 2 Stunden bei 4° inkubiert und anschließend die Platten ausgewertet. Bei vorhandener Hämagglutininreaktion werden die Erythrozyten daran gehindert, an den konischen Gefäßwänden der Mikrotiteröffnungen zu Boden zu sinken, was durch diffuse Rotfärbung des gesamten Bereiches angezeigt wird.

4.  Herstellung des Enterotoxin-produzierenden Bakterienstammes E.coli K12 5K/pEFK700 + pRK248cIts

4.1. Isolierung und Charakterisierung der Plasmide pHUB2 sowie pRK248cIts aus E.coli Stämmen

Man verfährt analog wie unter 1.1. beschrieben. Die auf die beschriebene Weise hergestellte DNA erweist sich als frei von chromosomalen Verunreinigungen.

4.2.  Ligierung der Ent-LT Genregion mit dem Vektorplasmid pHUB2

Das in das Ent-Plasmid pEFK295 integrierte DNA-Fragment mit einer relativen Molekülmasse von 5,8 Md (8,78 kb) wird mit dem Transkriptionsvektor pHUB2 ligiert. Das Vektorplasmid pHUB2 ist in der Literatur beschrieben (Bernard et. al, Gene 5, 1979, 59-76) und trägt eine vom Bakteriophagen $\lambda$ stammende Promotorregion mit der Bezeichnung $p_L$. Der $p_L$-Promotor wird durch das Genprodukt des ebenfalls im Phagengenom vorhandenen cI Gens negativ reguliert und stellt in Zusammenwirkung mit weiteren Phagengenprodukten einen genetischen Schaltmechanismus dar, durch den Lysogenität bzw. Induktion des Bakteriophagen $\lambda$ innerhalb der E.coli Wirtsstelle bestimmt werden. Es ist bekannt, daß $p_L$ ein sehr effektiver Promotor mit hoher Transkriptionsrate ist.

Bei der Ligierungsreaktion von 3,5 µg pHUB2-DNA mit 12,5 µg pEFK295-DNA wird, wie unter 1.2 beschrieben, vorgegangen.

4.3.  Transformation von E.coli Zellen mit dem Plasmid pRK248cIts

E.coli K12 5K Zellen werden zunächst mit dem Plasmid pRK248cIts transformiert, um die Stabilität der anschließend zu transferierenden $p_L$-tragenden Plasmidrekombinanten innerhalb der Bakterienzellen zu gewährleisten. Als Selektionsplatten finden Blutagarplatten mit je 30 µg/ml Tetracyclin Verwendung. Auf diesen Platten sind nur Transformanten zum Wachstum befähigt, d.h. Bakterien, die Plasmid-DNA aus der Lösung aufgenommen haben und deren Plasmide auch in den Keimen repliziert werden können.

4.4.  Transformation von E.coli K12 5K/pRK248cIts Zellen mit der in vitro rekombinierten DNA

Die Transformation von E.coli K12 5K/pRK248cIts Keimen mit dem Plasmid pEFK700 erfolgt wie unter 4.3 beschrieben. Nach Selektion auf Tetracyclin- und Kanamycinresistenz werden alle Rekombinanten im analy-

tischen Minilysatverfahren untersucht und ihre Plasmid-DNA in Agarosegelen charakterisiert. Dabei wird, wie unter 1.4 beschrieben, vorgegangen.

Restriktionsverdauung mit der Restriktionsendonuklease BamHI und Agarosegelelektrophorese werden anschließend, wie beschrieben, durchgeführt. Auf diese Weise identifizierte Bakterienklone enthalten ein rekombiniertes Plasmid, welches ein BamHI Restriktionsfragment mit einer relativen Molekülmasse von 5,8 Md (8,78 kb) enthält. Aus diesen Keimen wird die Plasmid-DNA durch Cäsiumchlorid-Dichtegradientenzentrifugation in Gegenwart von Äthidiumbromid, wie beschrieben, isoliert und neuerlich durch Spaltung mit verschiedenen Restriktionsendonukleasen charakterisiert. Jene Keime, deren rekombinante Plasmide der in Abb. 6 angegebenen Genkarte entsprechen, werden auf die Synthese für hitzelabiles Enterotoxin geprüft.

4.5. Test der durch in vitro-Genrekombination erzeugten Bakterienklone auf Enterotoxinproduktion im passiven Immunhämolysetest

Als Test auf gebildetes Ent-LT findet das beschriebene passive Immunhämolyseverfahren Verwendung. Die Hämolysetests ergeben für pEFK700 tragende Keime die in Tab. 5 dargestellten Werte. Die Gesamtsynthese von hitzelabilem Enterotoxin liegt in den durch Genrekombination hergestellten E.coli Keimen um einen Faktor von etwa 80-100 höher als im entsprechenden Wildtyp-Stamm E.coli 711/P307.

Aus den Ergebnissen der durchgeführten Restriktionsanalysen kann geschlossen werden, daß das als pEFK700 bezeichnete, in vitro rekombinierte Hybridplasmid das gesamte Strukturgen für hitzelabiles Enterotoxin trägt. Außerdem ist auch die Orientierung des klonierten BamHI-Fragments durch Restriktionsanalysen eindeutig festgelegt.

5. Herstellung des Enterotoxoid-produzierenden Bakterienstammes E.coli K12 5K/pEFK800 + pRK248cIts

5.1   Gezielte Entfernung des für die Enteropathogenität verantwortlichen Ent-LT Genanteils aus dem Plasmid pEFK700 und Vorschalten des $p_L$-Promotors vor das Ent-LT-B Gen

Der gewählte experimentelle Weg besteht in der enzymatischen Öffnung des Plasmids pEFK700 mit der Restriktionsendonuklease EcoRI und anschließendem Ringschluß des größeren der dabei entstehenden Fragmente durch Ligierung mit T4-Ligase. Um zu gewährleisten, daß eine solche Deletion mit erhöhter Wahrscheinlichkeit stattfindet, werden 2,5 µg gereinigter pEFK700-DNA mit EcoRI vollständig gespalten und anschließend in einem Volumen von 150 µl ligiert. Sämtliche anderen experimentellen Bedingungen für Restriktionsverdauung, Phenolextraktion, DNA-Fällung und Ligierung bleiben unverändert und entsprechen der zur Herstellung des Hybridplasmids pEFK700 beschriebenen Vorgangsweise. Nach Transformation kompetenter E.coli K12 5K/pRK248cIts Zellen mit der im Ligasegemisch anwesenden DNA werden auf die beschriebene Weise alle jene Transformanten selektioniert, welche Kanamycin- und Tetracyclinresistenz aufweisen. Aus diesen Keimen wird die Plasmid-DNA isoliert und die beabsichtigte Deletion im neu entstandenen Plasmid mit der Bezeichnung pEFK800 durch vergleichende Spaltung der Plasmide pEFK700 und pEFK800 mit den Restriktionsendonukleasen EcoRI, BamHI und HindIII eindeutig bestätigt. Es liegt somit ein durch in vitro- Genrekombination künstlich hergestelltes Plasmid vor, welches die genetische Information für die bindende Proteinuntereinheit des hitzelabilen E.coli Enterotoxins besitzt. Jene DNA-Region, welche für die A-Untereinheit des Enterotoxins, die für die Toxizität verantwortlich ist, kodiert, ist mit Hilfe des beschriebenen Deletionsexperiments aus dem Plasmid pEFK700 entfernt worden.

Eine korrekte Transkription des verbleibenden Enterotoxingenfragments ist vom beschriebenen, sehr effektiven $p_L$-Promotor des Vektorplasmids zu erwarten, wobei vorauszusetzen ist, daß der korrekte Leserahmen ebenfalls erhalten geblieben ist. Tatsächlich ist ein im Anschluß an das Klonierungsexperiment durchgeführter passiver Immunhämolysetest für pEFK800 tragende E.coli Keime positiv. Unter geeigneten Züchtungsbedingungen (vgl.

Tab. 6) liegen die Ausbeuten um einen Faktor von etwa 200-250 höher als im entsprechenden Wildtypstamm E.coli 711/P307.

5.2    In vitro-Toxizitätstest der durch in vitro-Genrekombination hergestellten Bakterienklone

Um zu bestätigen, daß es sich bei pEFK800 tragenden Keimen um Bakterienklone handelt, die imstande sind, ein atoxisches, aber immunologisch aktives Enterotoxoid zu synthetisieren, wird ein in vitro-Toxizitätstest an adrenalen Y1-Zellen durchgeführt. Neben Choleratoxin-als positiver und plasmidfreien E.coli K12 5K/pRK248clts Keimen als negativer Kontrolle wird der Wildtyp-Enterotoxinproduzentenstamm E.coli 711/P307 neben den durch in vitro-Genrekombination erhaltenen Stämmen E.coli -K12 5K/pEFK700 + pRK248clts und E.coli K12 5K/pEFK800 + pRK248clts eingesetzt.

Die Auswertung des Y1-Tests ergibt, daß nur P307 bzw. pEFK700 tragende Keime die für die Enterotoxineinwirkung charakteristischen morphologischen Veränderungen ergeben. Die mit E.coli K12 5K/pEFK800 + pRK248clts behandelten Y1-Zellen zeigen keine morphologischen Verände-rungen.

5.3    Abhängigkeit der Enterotoxin- (bzw. -toxoid-) Produktion von den
       Wachstumsbedingungen der Keime

Entsprechend den aus der Literatur (Bernard et al, Gene $\underline{5}$, 1979, 59-76) bekannten Daten sind zur optimalen Ausnützung des $p_L$-Promotors bestimmte Züchtungsbedingungen der beschriebenen Bakterienklone notwendig. Demnach sollen die Keimsuspensionen zunächst bei Temperaturen um ca. 30° (abgeschalteter $p_L$-Promotor) zu möglichst hohen Zelldichten herangezogen werden. Nach kurzem Bebrüten (ca. 30 Minuten) bei 45° soll anschließend der $p_L$-Promotor aktiviert werden. Die unter Kontrolle des $p_L$-Promotors ablaufende Genexpression soll sodann bei einer Temperatur von 42° für mehrere Stunden ablaufen. Antibiotikazusatz während der Expressionsphase soll vermieden werden.

Die Ent-LT Syntheserate pEFK700 tragender Keime ist in einem Züchtungstemperaturbereich von 32 bis 42° weitgehend konstant. Die zu erwartende Abhängigkeit der Toxoidausbeute von den Züchtungsbedingungen tritt nur bei Keimen mit dem Plasmid pEFK800 ausgeprägt zutage. Die genauen Angaben über Züchtungsbedingungen sowie erzielte Ausbeuten sind in Tabelle 6 zusammengefaßt. Aufgrund der relativ großen Distanz der $p_L$-Promotorregion vom Ent-LT Strukturgen im Plasmid pEFK700 scheint dieser Promotor auf die Expression des Enterotoxin-Gens wenig Einfluß auszuüben. Die Transkription des Ent-LT Gens erfolgt wahrscheinlich vornehmlich vom eigenen Ent-LT Promotor aus (vgl. Abb. 6).

5.4  Transformation von Haftantigen-synthetisierenden Bakterien mit den Plasmiden pRK248clts sowie pEFK700 bzw. pEFK800

Für eine Vakzine ist neben dem von pEFK700 oder pEFK800 kodierten Toxin bzw. Toxoid die Biosynthese eines Haftantigens erforderlich. Die Herstellung solcher Bakterien gelingt durch Transformation von E.coli K12 5K Zellen, die bereits für ein schweinespezifisches K88-Antigen kodierende Plasmide tragen, mit den Plasmiden pRK248clts sowie pEFK700 bzw. pEFK800. E.coli K12 Stämme, die porcines Haftantigen des Serotyps K88(AB) produzieren, stammen von P.Guinee, National Institute of Health, Bilthoven, Niederlande. Die Transformation dieses Stammes mit 1 μg des Plasmids pRK248clts erfolgt auf die beschriebene Weise. Nach anschließender Transformation von E.coli K12 5K/K88(AB) + pRK248clts Keimen mit pEFK700 bzw. pEFK800 durchgeführte Plasmid-DNA Präparationen und entsprechende Restriktionsanalysen zeigen, daß Haftantigen-, Repressor- und Enterotoxinplasmide innerhalb jeweils einer Bakterienzelle stabil sind. Diese Stabilität bleibt auch nach einigen Phasen (mehr als 250 Generationszeiten) in antibiotikafreiem Medium erhalten.

Die Produktion des Enterotoxoids in Anwesenheit des Repressor- sowie K88-Plasmids wird im passiven Immunhämolysetest, der wie beschrieben durchgeführt wird, bestätigt. Auch die Synthese des K88-Antigens wird durch die gleichzeitige Anwesenheit von Repressorplasmid sowie pEFK700 oder von pEFK800 in der Zelle nicht beeinflußt, wie im Hämagglutinationstest in Gegenwart von Mannose gezeigt werden kann. Der Test wird wie beschrieben durchgeführt.

Alle beschriebenen K88-Haftantigenplasmid tragenden Keime zeigen mannoseresistente Hämagglutination an Hühnererythrocyten.

6.    Herstellung    des    Enterotoxoid-produzierenden    Bakterienstammes E.coli K12 5K/pEBK620

6.1  Isolierung des Plasmids pKS100

Ca. 1 ml der angezüchteten Übernachtkultur von E.coli F165Δ(gal)+rp/ pKS100 wird in Eppendorf-Röhrchen zentrifugiert, der Überstand mit einer Pasteurpipette abgesaugt und die Zellen in je 100 µl Lysozymlösung suspendiert. Die Lysozymlösung hat folgende Zusammensetzung: 20 mg Lysozym, 500 µl 1 M Glukose, 10 ml 0,1 M Na$_2$·EDTA, 250 µl 1 M Tris-HCl, pH = 8,0. Nach 30-minütigem Stehen im Eis erfolgt ein Zusatz von 200 µl einer Lösung, die durch Zusammenmischen von 2 ml einer 1 M Natriumhydroxidlösung, 2 ml einer 5 %igen SDS-Lösung und 5,5 ml destillierten Wassers erhalten wird. Nach 5 Minuten weiteren Stehens im Eis werden 150 µl einer 3 M Natriumazetatlösung, pH = 4,8, zugesetzt. Das Gemisch wird weitere 60 Minuten im Eisbad belassen und anschließend 5 Minuten in einer Eppendorf-Zentrifuge zentrifugiert. 400 µl des zentrifugierten Überstandes werden schließlich in ein neues Eppendorf-Röhrchen überführt und die DNA mit 1 ml 96 %igem Äthanol bei -20° 30 Minuten lang gefällt. Nachdem zwei weitere Male auf die beschriebene Weise umgefällt wurde, wird das DNA-Pellet 15 Minuten im Vakuumexsikkator getrocknet, anschließend in 40 µl destilliertem Wasser gelöst und mit 2 µl pankreatischer RNase (1 mg/ml in 5 mM Tris-HCl, pH = 8,0) (Serva, Heidelberg) 30 Minuten lang bei 37° inkubiert.

Es folgt ein Ausschütteln mit wassergesättigtem, auf neutralen pH gebrachtem Phenol, die wäßrige Phase wird nach Zentrifugation und Trennung der Phasen 4 x mit Diäthyläther ausgeschüttelt, und die DNA erneut durch Zugabe von 2 Teilen 96 %igem Äthanol in 0,3 M Natriumazetat, pH 6,8, bei -20° für 30 Minuten gefällt. Der zentrifugierte DNA-Niederschlag wird kurz im Vakuum getrocknet.

6.2   Restriktionsverdauung der Plasmide pEFK295 und pKS100 mit der Restriktionsendonuklease HindIII, Phosphatasebehandlung und Reinigung der DNA über eine Mulekularsiebsäule

1 µg der Plasmid-DNA werden in jeweils 50 µl eines HindIII-Puffers (10 mM Tris-HCl, pH 7,4, 50 mM NaCl, 10mM MgCl$_2$, 15 mM DTT) zwei Stunden bei 37° verdaut. Der Probe mit der pKS100 DNA wird danach 1 µl Kälberdarmphosphatase zugesetzt und zwei weitere Stunden bei 37° inkubiert. Nach Beendigung der Inkubation werden beide Proben getrennt mit je 50 µl Wasser aufgefüllt und mit 100 µl wassergesättigtem, neutral gepuffertem Phenol ausgeschüttelt, zentrifugiert und die Überstände getrennt über eine 3 ml Sephadex G100-Säule in eine Pasteurpipette filtriert. Die Säule ist mit einem Puffer aus 10 mM Tris-HCl, pH 8,0 + 0,1 mM EDTA äquilibriert. Eluiert wird mit demselben Puffer, und 200 µl Fraktionen werden in Eppendorf-Gefäßen aufgefangen. Die Fraktionen Nr. 4-6 enthalten die gereinigte DNA. Diese wird nach Zusatz von 20 µl 3 M Natriumazetat, pH 6,0, und 600 µl 96 %igem Äthanol bei -20° für 30 Minuten gefällt. Nach Sedimentierung der DNA durch Zentrifugation in der Servall-Zentrifuge bei 19.000 upm für 20 Minuten wird der Äthanol vorsichtig abgehebert und die DNA im Vakuum kurz getrocknet.

6.3   Ligierung der HindIII-DNA-Fragmente von pEFK295 mit der HindIII verdauten DNA von pKS100

Die, wie unter 6.2 beschrieben, gereinigten HindIII-Spaltprodukte von pEFK295 und pSK100 werden im Verhältnis 1 µg : 0,5 µg gemischt, in 20 µl eines T4-DNA-Ligase-Puffers gelöst und mit 2 µl T4-Ligase über Nacht im Kühlschrank inkubiert.

Die Zusammensetzung des T4-DNA-Ligase-Puffers ist wie folgt: 30 mM Tris-HCl, pH 8,0, 4 mM MgCl$_2$, 1,2 mM EDTA, 0,2 mM ATP, 10 mM DTT, 50 µg/ml BSA.

6.4   Transformation von E.coli Zellen mit der in vitro-rekombinierten DNA

Das Ligasegemisch wird direkt für die Transformation kompetenter Zellen eingesetzt. Kompetente Zellen werden wie folgt hergestellt: 50 ml L Broth (10 g Bacto Trypton, 5 g Bacto Yeast Extract, 2 g NaCl, 2 ml NaOH, 10 ml 20 %ige Glukoselösung und 1 l Wasser) werden mit Zellen von E.coli K12 F 165 $\Delta$ (gal) trp angeimpft, bei 37° bis zu einer optischen Dichte von 0,3 bei 546 nm und 1 cm Schichtdichte anwachsen gelassen und dann 10 Minuten bei 6000 upm im SS 34-Rotor der Scrvall-Zentrifuge zentrifugiert. Das Zellpellet wird 2 x mit eiskaltem CaCl$_2$ (100 mM) gewaschen und zwischen den Waschvorgängen jeweils 10 Minuten bzw. 20 Minuten in Eis inkubiert, ehe es erneut abzentrifugiert wird. Das Zellpellet wird schließlich in 1,7 ml 100 mM eiskaltem CaCl$_2$ suspendiert und erneut 30 Minuten in Eis inkubiert. 0,2 ml dieser Zellsuspension werden mit 0,1 µg DNA des Ligasegemisches in 100 mM CaCl$_2$ 30 Sekunden bei 37°, dann 90 Minuten in Eis, weitere 3 Minuten bei 45° inkubiert und dann 1 Stunde bei 37° in 1 ml dYT-Medium in einem 100 ml Erlenmeyer-Kolben wachsen gelassen. Das dYT-Medium hat folgende Zusammensetzung: 0,5 % NaCl, 1,6 % Bacto Trypton, 1 % Bacto Yeast Extract, 0,4 % Glukose (alles in Wasser). Je 0,2 ml der Zellanzucht in dYT werden auf Nutrient-Agarplatten mit 50 µg/ml Ampicillin plattiert. Ampicillinresistente Kolonien werden selektioniert.

6.5   Charakterisierung des Enterotoxoidplasmids pEBK620 mit Hilfe von Restriktionsendonukleasen und durch DNA-Sequenzierung

Von ca. 50 der unter 6.4 isolierten ampicillinresistenten Zellklonen wird die Plasmid-DNA, wie unter 6.1 beschrieben, isoliert und die DNA, wie unter 6.2 beschrieben, mit HindIII gespalten. Die Spaltstücke werden an Sephadex G100 gereinigt (vergl. 6.2) und in einem Agarose-Äthidiumbromid-Gel elektrophoretisch aufgetrennt. Als Vergleich in der Elektrophorese dienen HindIII-Spaltstücke der pEFK295-DNA. Die Agarosegele werden wie folgt hergestellt: 1,6 g Seakem Agarose werden nach Zusatz von 8 µl Äthidiumbromidlösung (10 mg/ml) in 160 ml TBE-Puffer (21,6 g Tris-OH p.a., 11 g Borsäure p.a., 1,86 g $Na_2$.EDTA, ad 2 l $H_2O$) aufgeschwemmt und am Rückflußkühler unter kräftigem Rühren 15 Minuten lang gekocht. Die vertikale Gel-Elektrophoreseapparatur der Fa. E - C Apparatus Corp. (St. Petersburg, Florida, USA) wird mit der auf 50° abgekühlten gelösten Agarose gefüllt. Ein Geltaschenformer wird eingesetzt und die Agarose durch Wasserkühlung zum Erstarren gebracht. In die erstarrte Geltasche wird die DNA-Probe durch Unterschichten eingefüllt. Die ca. 0,5-1 µg DNA werden dazu in 10-25 µl einer 40 %igen Saccharoselösung mit 0,1 M $Na_2$.EDTA, 0,05 % Bromphenolblau und 0,5 %igen Xylenecyanol aufgenommen. Die Elektrophorese wird bei 10V/cm in TBE-Puffer durchgeführt, und die DNA-Banden werden durch UV-angeregte Fluoreszenz sichtbar gemacht.

Die rekombinante DNA des Plasmids pEBK620 enthält - wie ein Größenvergleich der von ihr erhaltenen HindIII-Fragmente mit denen des Plasmids pEFK295 zeigt -zusätzlich zur Vektorplasmid-DNA des pKS100 das 780 bp lange HindIII-DNA-Fragment mit dem Gen für das E.coli hitzelabile Enterotoxoid und drei weitere HindIII-Fragmente aus pEFK295 mit 111 bp, 196 und 196 pb Länge. Die Anordnung dieser 4 in das pKS100 integrierten DNA-Fragmente relativ zueinander wird durch weitere Spaltungen der DNA mit verschiedenen anderen Restriktionsendonukleasen und durch DNA-Sequenzanalyse nach Maxam und Gilbert (1977, Proc. Natl. Acad. Sci. USA 74, 560) ermittelt. Dazu muß die Plasmid-DNA in größeren Mengen hergestellt werden: Der Stamm F 165$\Delta$(gal) trp / pEBK620 wird über Nacht in dYT-Medium angezogen. Je 500 ml M9K-Glukosemedium (0,7 % $Na_2HPO_4$.$2H_2O$, 0,3 % $KH_2PO_4$, 0,1 % $NH_4Cl$, 0,6 % Casamino acids (Difco), 1 ml 1M $MgSO_4$/l, 1 ml 1M $CaCl_2$/l, 5 ml $10^{-4}$M $FeCl_3$/l, 20 ml 20 %ige Glukose, 1 ml 0,1 % Thiamindichlorid) werden mit 20 ml Übernachtkultur in 2 l Erlenmeyer-Kolben angeimpft. Die Zellen werden im Schüttelbad bei 30° bis zur optischen Dichte von 1 bei 546 nm und 1 cm Schichtdicke unter starkem Schütteln anwachsen gelassen. Es werden 250 µg/ml Chloramphenicol dazugegeben, und die Kultur wird weitere 15 Stunden bei 30° kräftig geschüttelt. Anschließend werden die Zellen 10 Minuten bei 4000 upm im GSA-Rotor der Sorvall-Zentrifuge sedimentiert, und das Sediment je 1 l Kultur 2 x mit 50 ml M9-Puffer gewaschen (M9-Puffer: 0,7 % $Na_2HPO_4.2H_2O$, 0,3 % $KH_2PO_4$, 0,1 % $NH_4Cl$). Das Sediment pro 1 l Kultur wird in 10 ml 25 %iger Saccharose-Lösung in 50 mM Tris-HCl, pH 8 + 15 µg Lysozym aufgenommen und 10 Minuten in Eis gerührt. Dann werden 4 ml 0,25 M $Na_2$.EDTA, pH 8,5, dazugegeben und nach weiteren 15 Minuten Rühren in Eis noch 17 ml Doc-Detergenz (1 % Brij 58, 0,4 % Na-desoxycholat, 0,05 M Tris-HCl, pH 8, 0,0625 M EDTA). Nach 40 Minuten Inkubation in Eis wird 40 Minuten bei 19.000 upm im SS 34-Rotor der Sorvall-Zentrifuge zentrifugiert und der klare Überstand für die Weiterverarbeitung abgegossen.

Die erste Reinigung der Plasmid-DNA erfolgt in einem CsCl-Gleichgewichtsgradienten. Zu 15,5 ml Überstand werden 3 mg Äthidiumbromid und 15 g CsCl gegeben. Das Lysat wird in Polyallomer-Röhrchen bei 23.000 upm und 15° 72 Stunden im Rotor 60 Ti der Beckman-Ultrazentrifuge zentrifugiert. Die untere, bei langwelligem UV-Licht von 350 nm sichtbare Bande enthält die Plasmid-DNA. Die untere Bande wird ausgetropft. Es schließt sich ein weiterer CsCl-Gleichgewichtsgradient an. Die ausgetropfte Plasmidbande wird wieder in ein entsprechendes Zentrifugenröhrchen gegeben und mit einer Lösung von 3 mg Äthidiumbromid + 15 g CsCl pro 15 ml TE-Puffer (10 mM Tris-HCl, pH 8, 1 mM EDTA) aufgefüllt. Es wird erneut zentrifugiert - diesmal 48 Stunden bei 48.000 upm und 15° im Festwinkelrotor 50 Ti - und die untere Bande wieder unter UV ausgetropft. Um die Lösung weitgehend zu entsalzen, wird sie in Dialyseschläuche gefüllt und 3 x gegen 2 l TE-Puffer dialysiert. Die Dialysedauer beträgt ca. 3 Stunden.

Um das Äthidiumbromid aus der DNA zu entfernen, wird die Lösung ca. 3 x phenolisiert. Zu der Probe wird ein Volumen neutrales Phenol (d.h. an 0,5 M Tris, pH 8, gesättigtes Phenol) gegeben, die Lösung wird am Vortex geschüttelt und kurz zentrifugiert. Es bilden sich zwei Phasen, wobei sich das mehr lipophile Äthidiumbromid in der unteren Phenolphase, die Plasmid-DNA in der wäßrigen Phase befindet. Die obere wäßrige Phase mit der Plasmdid-DNA wird abpipettiert und so lange phenolisiert, bis sie farblos ist, d. h. bis sich das gesamte, in die DNA interkalierte Äthidiumbromid in der Phenolphase befindet.

Die so von CsCl und Äthidiumbromid gereinigte Lösung wird in Corex-Zentrifugenröhrchen pipettiert, und dann 1/10 Volumen an 3 M Natriumazetat und das 2,5-fache Volumen an Äthanol dazugegeben. Die Fällung der DNA erfolgt 1 Stunde bei -20°. Anschließend wird die Probe 40 Minuten bei 9.000 upm zentrifugiert. Das Sediment wird in ca. 200 µl 0,3 M Natriumazetat resuspendiert, in ein Eppendorf-Gefäß überführt und erneut mit Äthanol gefällt. Die Zentrifugation erfolgt jetzt 20 Minuten bei 19.000 upm. Die DNA wird dann im Vakuum kurz getrocknet.

Die Konzentration der DNA wird photometrisch bei einer Wellenlänge von $\lambda = 260$ nm bestimmt. Es entspricht dabei 1 µg/ml Doppelstrang-DNA einer optischen Dichte von 0,02 bei einer 1 cm Küvette.

### 6.5.1 Spaltung der gereinigten DNA mit der Restriktionsendonuklease HindII

1 µg der pEBK620 DNA wird mit 21 Einheiten HindII bei 37° 2 Stunden in 25 µl 10 mM Tris-HCl, 50 mM NaCl, 10 mM $MgCl_2$, 10 mM DTT gespalten, anschließend gereinigt, wie unter 6.2 beschrieben, und in einem Agarosegel, wie oben beschrieben, aufgetrennt. Es entstehen 5 DNA-Stücke der Längen 3255 bp, 3250 bp, 1450 bp, 795 bp und 300 bp. Die HindII-DNA-Fragmente der Längen 3255 bp, 3250 bp, 1450 bp und 300 bp stammen aus dem pKS100-Plasmid bzw. dem Ent-LT-B-Gen, wie aus den aus der Literatur bekannten Schnittstellen für HindII in pBR322 (J.G. Sutcliff [1978], Nucl. Acid Res., 5, 2721), dem Gal-Operon (Trinks et al. [1981], Molec. Gen. Genet. 182, 183) und dem Ent-LT-B-Gen (W.S. Dallas, S.E. Falkow [1980],

Nature 288, 499, E.K. Spicer et al. [1981], Proc. Natl. Acad. Sci., USA 78, 50) geschlossen werden kann. Das 795 bp lange HindII-Fragment enthält demnach die Integrationsstellen der in pEBK620 vorhandenen 4 HindIII-Fragmente. Für die weitere Analyse wird das 795 bp lange HindII-Fragment in präparativem Maßstab gewonnen: 100 µg pEBK620 DNA werden in 200 µl HindII-Puffer mit 200 E HindII-Enzym 2 Stunden bei 37° gespalten, phenolisiert, über eine Sephadex-G100-Säule gereinigt und mit Äthanol gefällt, wie unter 6.2 beschrieben. Die gefällte und gereinigte DNA wird in einem Agarose-Äthidiumbromidgel elektrophoretisch aufgetrennt. Die Elektrophorese erfolgt wie oben beschrieben, doch wird hier eine horizontale Gelapparatur der Fa. Bethesda Research Laboratories (Bethesda, Maryland, USA) und eine niedrig schmelzende Seaplaque Agarose (Marine Colloids, Rockland, Maine, USA) benutzt. Diese Agarose erlaubt es, DNA auf eine einfache Weise zu eluieren:

Die DNA-Banden werden unter langwelligem UV-Licht sichtbar gemacht. Das Agarosegelstück, das die 795 bp lange DNA-Bande enthält, wird mit einer Rasierklinge ausgeschnitten, mit 2 Volumen 0,05 M Tris-HCl, pH 8, und 0,5 mM EDTA versetzt und 3 Minuten auf 65° erhitzt. Dabei schmilzt die Agarose, ohne daß die DNA denaturiert. Die Lösung wird auf 37° abgekühlt, 2 x mit wassergesättigtem neutralem Phenol ausgeschüttelt und zentrifugiert. Der wäßrige Überstand wird mit Diäthyläther ausgeschüttelt und der restliche Äther durch Inkubation der Probe bei 37° entfernt. Anschließend wird die DNA nach Zugabe von 1/10 Volumen 3 M Natriumazetat und 2 Volumen 96 %igem Äthanol bei -20° gefällt. Die DNA wird 40 Minuten bei 9.000 upm in Glas-Corex-Röhrchen der Sorvall-Zentrifuge zentrifugiert. Für eine zweite Äthanolfällung wird die DNA eines Corex-Röhrchens in 500 µl 10 mM Tris-HCl, 0,1 mM EDTA, pH 8, gelöst, auf 2 Eppendorf-Röhrchen verteilt, mit 25 µl 3 M Natriumazetat und 750 µl 96 %igem Äthanol versetzt und erneut bei -20° inkubiert. Nach Zentrifugation in der Sorvall-Zentrifuge im SS 20-Rotor für 20 Minuten bei 19.000 upm und Entfernen des Äthanols wird die DNA kurz im Vakuum getrocknet.

6.5.2    Spaltung des 795 bp HindIII-Fragments aus pEBK620 mit weiteren Restriktionsenzymen zur Aufstellung eiener physikalischen Genkarte

Für die elektrophoretische Auftrennung der nach Restriktionsspaltung erhaltenen DNA-Fragmente wird ein Acrylamidgel verwendet: Dazu wird eine Apparatur für 20 x 40 cm Gele benutzt. Das Gel wird zwischen 2 20 x 40 cm Glasplatten gegossen, von denen eine einen Einschnitt für den Taschenformer hat. Die Schichtdicke ist 1,2 mm. Die Glasplatten werden an den Seiten mit einer 1 %igen Agaroselösung und am unteren Ende mit Klebeband abgedichtet. Während die kleineren Fragmente (50-200 bp) ein gutes Bandenmuster auf 10 und 12 % Polyacrylamidgelen zeigen, trennen sich Banden höhermolekularer DNA besser in 4 und 6 % PA-Gelen.

Gelansatz:

$X$ % Acrylamid (mit $X = 4, 6, 10, 12$)

1/30 $X$ % Bisacrylamid 20 µl/100 ml N,N,N',N'-Tetramethyläthylendiamin in TBE-Puffer

Die Lösung wird filtriert und an der Wasserstrahlpumpe entgast. Vor dem Gießen des Gels wird noch 0,05 % Ammoniumperoxid dazugegeben. Nach etwa 2 Stunden ist das Gel polymerisiert. Der Taschenformer wird vorsichtig herausgezogen und die Geltaschen gut mit Puffer nachgespült, um eventuell noch nicht polymerisiertes Acrylamid herauszuwaschen. Die DNA wird ca. 16 Stunden bei 100-200 V aufgetrennt. Nach Beendigung der Elektrophorese werden die DNA-Restriktionsfragmente durch Baden des Gels in 1 l Wasser mit 0,5 µg/ml Äthidiumbromid gefärbt und unter UV-Licht sichtbar gemacht.

Das 795 bp-Fragment wird mit den Restriktionsendonukleasen gespalten, die Spaltstücke auf das Acrylamidgel gegeben und elektrophoretisch aufgetrennt. Die Länge der einzelnen Fragmente wird durch einen Vergleich ihrer Wanderungsstrecke im Gel mit der Wanderungsstrecke von DNA-Fragmenten bekannter Länge ermittelt. Als Längenstandard dient ein HpaII-Verdauungsgemisch der Plasmid-DNA pBR322 (J.G. Sutcliffe [1978], Nucl.Acids Res. 5, 2721). Ein Vergleich der Längen der DNA-Stücke nach Einzelverdauung mit den Restriktionsendonukleasen HindIII, PstI oder HaeIII mit den Längen der DNA-Stücke nach Doppelverdauung mit HindIII + PstI und PstI + HaeIII erlaubt, eine Restriktionskarte des 795 bp HindII-Fragmentes aus pEBK620 zu erstellen (Abb. 9). Die Einzelverdauungen der DNA erfolgen, wie unter 6.2 für HindIII beschrieben; für die Doppelverdauungen wird wie folgt vorgegangen: 1 µg DNA in 25 µl Verdauungspuffer werden 2 Stunden bei 37° mit 2 Einheiten des 1. Enzyms inkubiert; dann werden 5 µl des 10-fach konzentrierten Puffers für das 2. Enzym zugegeben und 20µl Wasser und 2 Einheiten des 2. Enzyms. Es wird erneut 2 Stunden bei 37° inkubiert. Der PstI-Puffer hat die Zusammensetzung: 10 mM Tris-HCl, pH 7,4, 50 mM NaCl, 10 mM $MgCl_2$; der HaeIII-Puffer: 6 mM Tris-HCl, pH 7,4, 6 mM $MgCl_2$, 6 mM Mercaptoäthanol.

Aus der Restriktionskarte der pEBK620 DNA im Bereich der Integrationsstelle des Ent-LT-B-Gens ist ersichtlich, daß vor dieses Gen drei kleinere HindIII-Fragmente aus dem pEFK295-Plasmid integriert sind; 2 x ein Fragment von 196 bp Länge in identischer Orientierung, getrennt durch ein drittes Fragment von 111 bp Länge. Die drei zusätzlichen Fragmente proximal zum Ent-LT-B-Gen werden für die Überproduktion des Toxoids in Stämmen mit diesem Plasmid verantwortlich gemacht, da E.coli Stämme mit pKS100 Ent-LT-B-Plasmiden, die diese zusätzlichen Fragmente nicht oder in anderer Anordnung enthalten, weniger Toxoid produzieren.

6.5.3    DNA-Sequenzanalyse des 795 bp HindII-Segmentes aus pEBK620

Zur Absicherung der Restriktionskarte des 795 bp DNA-Stückes aus pEBK620 wird die DNA nach der Methode von Maxam und Gilbert [1977], Proc. Natl. Acad. Sci. USA, 75, 560, sequenziert:

a) Phosphatase-Behandlung:

Durch die Behandlung mit alkalischer Phosphatase werden die Phosphat

gruppen an den 5'-Enden der DNA-Fragmente entfernt. Es wird soviel DNA eingesetzt, daß eine für die Kinasereaktion günstige Konzentration an 5'-Enden von 50 pMol entsteht. Der DNA in der Lösung mit Restriktionsenzym und Puffer werden 2 µl alkalische Phosphatase (2,5 E/l) zugesetzt und 1 Stunde bei 37° inkubiert. Die Reaktion wird durch Zugabe von 5 µl 0,1 M EDTA und Erhitzen von 5 Minuten auf 65° terminiert. Die DNA wird 2 x phenolisiert. Die wäßrigen Überstände werden nach Phasentrennung vereinigt und über eine G100-Säule gereinigt. Die gereinigte DNA wird wieder mit Äthanol gefällt und nach Zentrifugation kurz im Vakuum getrocknet.

b) Kinase-Reaktion:

Mittels der Polynukleotidkinase und $\gamma^{32}$P rATP werden die abgespaltenen Phosphatgruppen durch radioaktiv markierte Phosphatgruppen ersetzt. Die getrockneten DNA-Fragmente werden in insgesamt 13 µl PNK-Puffer (PNK: 0,4 M Tris-HCl, pH 9, 0,1 M $MgCl_2$, 10 mM DTT, 50 % Glycerin) aufgenommen und dann überführt in 35 µl evaporiertes $\gamma^{32}$P rATP (ca. $2.10^{-5}$ Mol und 0,12 mCi).

Der in 13 µl PNK-Puffer gelösten DNA mit den 35 µl eingetrockneten $\gamma^{32}$P rATP werden 2 µl Polynukleotidkinase (10E/µl, P-L Biochemicals, Milwaukee, Wisconsin, USA) zugesetzt, und es wird 28 Minuten bei 37° inkubiert. Dann kommen 1 µl 10 µM rATP dazu (verdrängt das heiße rATP aus dem Enzym), und es wird weitere 2 Minuten bei 37° inkubiert. Schließlich wird die Reaktion durch Zugabe von 2 µl 0,1 M EDTA terminiert. Die DNA wird dann, wie oben beschrieben, mit Äthanol gefällt und nach Zentrifugation im Vakuum kurz getrocknet.

c) Nachspaltung der markierten Fragmente und ihre Auftrennung im Polyacrylamidgel:

Die DNA wird nun mit einer geeigneten (Abb. 10) Restriktionsnuklease nachgespalten, sodaß die entstehenden Fragmente nur noch an einem der beiden 5'-Enden mit $^{32}$P markiert sind. Die Fragmente werden in einem 12 %igen PA-Gel aufgetrennt. Das nach der Elektrophorese in Frapanfolie gewickelte Gel wird mit radioaktiver Tinte markiert und auf einem Film (Kodak AR) ca. 30 Minuten exponiert. Die entsprechende DNA-Bande wird aus dem Film ausgeschnitten und der Film dann so auf das Gel gelegt, daß die Markierungen von Film und Gel übereinander liegen. Die Bande wird nun durch den Film ausgeschnitten und das DNA-Fragment aus dem Acrylamid eluiert. Die Gelstückchen werden dazu in 0,5-1 ml TE-Puffer aufgenommen und mit einem Glasstab fein zerbröselt. Die Suspension wird bei 45-65° über Nacht inkubiert. An nächsten Tag wird dann 2 Minuten in der Eppendorf-Zentrifuge zentrifugiert und die überstehende Flüssigkeit abpipettiert. Diese wird weiter wie oben phenolisiert, ausgeäthert und die DNA mit Äthanol gefällt, abzentrifugiert und getrocknet: Vor der Äthanolfällung wird das Volumen der Probe auf 800 µl eingestellt und 80 µl 3 M Ammoniumazetat und 8 µl ultrabeschallte Kalbsthymus-DNA (1 mg/ml) zugegeben. Die Proben werden auf 4 Eppendorf-Gefäße verteilt, die DNA mit Äthanol gefällt und anschließend kurz getrocknet.

d) Limitierte, basenspezifische chemische Spaltung der DNA:

Die Fragmente werden 4 parallelen chemischen Reaktionen unterworfen:

(1) G-spezifische Reaktion

Die Probe wird mit 200 µl Caco-Start Mix (50 mM Na-Cacodylat, 0,1 mM EDTA, 10 mM MgCl$_2$) vermischt, auf 0° gekühlt und mit 1 µl Dimethylsulfat 20 Minuten bei 20° inkubiert. Dann wird mit 50 µl Stop Mix (1,5 M Na-azetat, pH 7, 1 M Mercaptoäthanol) und 5 µl tRNA (1 mg/ml) beendet, 750 µl Äthanol zugesetzt und wie unter (5) weiterbehandelt.

(2) A-spezifische Reaktion

Die Probe wird mit 80 µl Wasser und 20 µl 5 N NaOH 30 Minuten bei 90° inkubiert, dann auf 0° gekühlt und mit 150 µl 1 N Essigsäure und 5 µl tRNA (1 mg/ml) versetzt. Dann werden 750 µl Äthanol zugesetzt und wie unter (5) weiterbehandelt.

(3) T + C-spezifische Reaktion

Die Probe wird mit 27 μl Wasser versetzt und im Eisbad auf 0° gekühlt. Dann wird mit 30 μl Hydrazinhydrat 40 Minuten bei 37° inkubiert und anschließend 200 μl 0,3 M Ammoniumazetat (1 mM an EDTA) und 5 μl tRNA (1 mg/ml) zugesetzt. Dann werden 750 μl Äthanol zugegeben und wie unter (5) weiterbehandelt.

(4) C-spezifische Reaktion

Die Probe wird mit 20 μl 5 M NaCl und 7 μl Wasser versetzt und auf 0° gekühlt. Dann wird mit 30 μl Hydrazinhydrat 40 Minuten bei 37° inkubiert und anschließend 200 μl 0,3 M Ammoniumazetat (1 mM an EDTA) und 5 μl tRNA (1 mg/ml) versetzt. Dann werden 750 μl Äthanol zugesetzt und wie unter (5) weiterbehandelt.

(5) Der für alle 4 Abbau-Reaktionen gemeinsame Teil

Nach der Äthanolfällung wird die DNA abzentrifugiert und der Überstand sorgfältig und möglichst vollständig abgenommen. Der Überstand wird in 250 μl 0,3 M Ammoniumazetat aufgenommen, 750 μl Äthanol zugesetzt und bei -20° gefällt. Dann wird abzentrifugiert, der Überstand möglichst vollständig abgenommen und das Sediment im Vakuum getrocknet. Es werden 100 μl 1 M Piperidin zugesetzt, das Eppendorf-Gefäß gut verschlossen und gemischt, dann wird 30 Minuten bei 90° inkubiert, anschließend auf 0° gekühlt und in der Gefriertrocknungsanlage einrotiert. Nun werden 20 μl 0,25 % Ammoniak zugesetzt, getrocknet, 20 μl Wasser zugesetzt, wieder getrocknet, 20 μl Wasser zugesetzt, wieder getrocknet und mit 3 μl Sequenzfarb-Mix (1 Volumen 1 mg/ml Bromphenolblau, 2 mg/ml Xylenecyanol FF, 2 mg/ml Orange G, 9 Volumen Formamid) 2 Minuten bei 90° aufs Gel aufgetragen.

Sequenzgel: Es wird eine 20 x 40 cm und eine 20 x 80 cm Gelapparatur benutzt. Die Trennkammer wird aus zwei 20 x 40 cm bzw. 20 x 80 cm Glasplatten, von denen eine einen Einschnitt für den Taschenformer hat, in einem Abstand von 0,4 mm zusammengebaut und mit Klebeband abgedichtet. Die Platte mit dem Einschnitt wird vorher auf der Innenseite silikonisiert (Platte mit einer Lösung von 2 % Dimethyldichlorsilan in Tetrachlor kohlenstoff oder Toluol befeuchten und bei 180° 3 Stunden trocknen).

Ansatz für die Acrylamidlösung:

X % Acrylamid von Bio Rad (mit X = 12,20)

1/20 X % Bisacrylamid ( " )

7 M Harnstoff

0,3 µl TEMED/ml

in TBE-Puffer.

Die fertige Lösung wird mit einer Spritze durch einen Steril-Filtervorsatz filtriert und an der Wasserstrahlpumpe entgast. Vor Gebrauch der Lösung werden noch 0,3 mg/ml Ammoniumperoxodisulfat dazugegeben.

Die Acrylamidlösung wird in die Trennkammer gefüllt und das Gel 1 Stunde polymerisiert. Nach einer Vorelektrophorese von 15 Minuten wird die Probe mit einer ausgezogenen 50 µl Kapillarpipette in die Geltasche gefüllt. Die Elektrophorese erfolgt zwischen 1200 und 2000 V. Anschließend wird das Gel mit Frapanfolie umwickelt und in einem Kodak AR Film bei -70° exponiert. Nach 3-12 Stunden Exposition kann der Film entwickelt und die DNA-Sequenz abgelesen werden. Für die vollständige Sequenzermittlung der beiden 196 bp HindIII-Fragmente und des 111 bp Fragments in pEBK620 werden diese Banden in das pBR322-Plasmid subkloniert. Die DNA des Plasmids pEBK620 wird dazu mit HindIII präparativ gespalten, wie unter 6.5.1 beschrieben. Doch werden hier die DNA-Fragmente nicht in Seaplaque Agarose, sondern in einem Saccharosegradienten aufgetrennt. Der Saccharosegradient wird in Polyallomer-Röhrchen für den Ausschwingrotor SW40 Ti hergestellt. Die Aufschichtung des linearen Gradienten erfolgt mit der Mischapparatur Peristaltic Pump P-3 (Pharmacia, Fine Chemicals). Pro Gradient werden je 7 ml 5 %ige und 25 %ige Saccharoselösung angesetzt (Saccharoselösung: Saccharose, gelöst in 10 mM Tris-HCl, pH 8, 1 mM EDTA, 100 mM NaCl (TES)-Puffer mit 1 % Äthidiumbromid). In das vordere Mischgefäß der Apparatur wird die 5 %ige Saccharoselösung, in das hintere Vorratsgefäß die höher konzentrierte Lösung gegeben, und die Röhrchen werden über eine 25 µl Kapillarpipette durch Unterschichten gefüllt. Ca. 100 µg DNA werden in 100 µl TES-Puffer aufgenommen und auf den Saccharosegradienten geschichtet. Der Gradient wird dann 16 Stunden bei 33.000 upm im Rotor SW40 Ti zentrifugiert. Die gesuchte Bande wird

unter langwelligem UV-Licht von 350 nm ausgetropft. Die DNA der unter UV ausgetropften Bande wird mit 1/10 Volumen 3 M Natriumazetat (pH 6) versetzt. Es folgen zwei Äthanolfällungen, an die sich zwei Phenolisierungen anschließen, um das Äthidiumbromid zu extrahieren. Das restliche Phenol wird durch 5-maliges Ausäthern der Probe entfernt. Die DNA wird erneut mit Äthanol gefällt, abzentrifugiert und im Vakuum kurz getrocknet.

Die beiden obersten im Saccharosegradienten sichtbaren Banden sind 196 bp und 111 bp lang, werden gemeinsam ausgetropft und mit Äthanol gefällt. Für die Reklonierung dieser Banden wird die Vektorplasmid-DNA pBR322 mit HindIII gespalten, mit Phosphatase behandelt und im Verhältnis 1 µg pBR322 zu 0,05 µg Fragment-DNA gemischt. Die Ligation der kleinen HindIII-Banden an pBR322, die Transformation und Selektion der transformierten Zellen geschieht genau so, wie unter 6.2, 6.3 und 6.4 beschrieben.

Die Plasmid-DNA ampicillinresistenter Klone wird, wie unter 2.1. beschrieben, isoliert, mit den Restriktionsendonukleasen HinfI bzw. HaeIII gespalten, und die Spaltprodukte werden in einem 6 %igen Polyacrylamidgel elektrophoretisch aufgetrennt. Da die HinfI bzw. HaeIII-Erkennungsstellen sowohl für pBR322 als auch für das 196 bzw. 111 bp lange Fragment bekannt sind (J.G. Sutcliffe [1978], Nucl. Acids. Res. 5, 2721, und Abb. 12), kann aus dem Spaltungsmuster sowohl auf die Anwesenheit als auch auf die Orientierung der integrierten kleinen HindIII-Fragmente in pBR322 geschlossen werden. Für die DNA-Sequenzierung werden 4 Klone ausgewählt, 2 mit dem kleineren HindIII-Fragment in beiden möglichen Orientierungen, zwei mit dem größeren HindIII-Fragment in beiden möglichen Orientierungen. Die Sequenzierungsstrategie ist in Abb. 11 wiedergegeben.

Zur DNA-Sequenzierung nach Maxam und Gilbert wird DNA der vier in Abb. 11 wiedergegebenen Plasmide, wie unter 6.1 beschrieben, isoliert, mit der Restriktionsendonuklease EcoRI gespalten, mit Phosphatase behandelt, gereinigt und $^{32}$P endmarkiert, wie oben beschrieben. Durch eine Spaltung der jeweiligen DNA mit der Restriktionsendonuklease BamHI werden die beiden markierten 5-Enden der DNA getrennt. Nach Elektrophorese in einem 6 %igen Acrylamidgel trennen sich 2 Banden, eine ist ca. 500 bp lang, die andere 3985 bp. Die längere entstammt dem pBR322 und wird verworfen, die kürzere enthält das zu sequenzierende Fragment in der einen oder anderen möglichen Orientierung. Die DNA des kürzeren Fragments wird aus dem Acrylamid eluiert und sequenziert. Die Ergebnisse der DNA-Sequenzierung sind in Abb. 12 wiedergegeben. Die Sequenz reicht von der HindII-Erkennungsstelle in dem Epimerasegen des Galaktoseoperons (galE) des pEBK620 bis zur HindII-Erkennungsstelle in dem Ent-LT-B-Gen (Abb. 9). Mit dieser Sequenz ist die für die besonders hohe Ausprägung des Ent-LT-B-Gens in pEBK620 verantwortliche DNA-Struktur vollständig aufgeklärt.

6.6    Transformation von E.coli K12 5K Zellen mit der pEBK620 DNA

pEBK620 Plasmid-DNA wird aus dem E.coli K12 F 165 Δ(gal) trp /pEBK620 Stamm gewonnen, wie unter 6.1 beschrieben. pEBK620 DNA wird benutzt, um Zellen von E.coli K12 5K zu transformieren, wie unter 6.4 beschrieben. Transformierte Zellen werden auf Nutrient Agar Platten mit 50 µg/ml Ampicillin selektioniert. Die pEBK620 DNA aus E.coli K12 5K wird durch Spaltung mit der Restriktionsendonuklease HindIII und anschließende Auftrennung der DNA-Stücke in einem Agarose-Äthidiumbromidgel, wie unter 6.2 beschrieben, charakterisiert. Ihr HindIII-Spaltungsmuster ist identisch mit der pEBK620 DNA aus E.coli K12 F Δ(gal) trp.

6.7    Test des durch in vitro-Genrekombination erzeugten Bakterienklons E.Coli K12 5K/pEBK620 auf Enterotoxoidproduktion im passiven Immunhämolysetest

Der isolierte Bakterienklon wird auf die Produktion von hitzelabilem Enterotoxoid geprüft. Als Test auf gebildetes Ent-LT findet das passive Immunhämolyseverfahren Verwendung, wie unter 1.5. bereits beschrieben wurde.

Schon nach den ersten Hämolysetests zeigt sich, daß in den Lysaten ultrabeschallter Zellen des Klons eine wesentlich höhere Menge an Entero- toxoid nachgewiesen werden kann, als dies in entsprechenden Zellysaten von E.coli K12 5K/pEFK295 der Fall ist. Ein quantitativer Vergleich der synthetisierten Enterotoxoidmenge in den Lyophilisaten des E.coliK- 12 5K/pEBK620 Keims zeigt, daß die Menge an hitzelabilem Enterotoxoid um einen Faktor von etwa 10 höher ist als die Toxinmenge im Stamm E.coli K12 5K/pEFK295.

6.8     Biologische Tests des durch in vitro-Genrekombination hergestellten Bakterienklons E.coli K12 5K/pEBK620.

Um zu bestätigen, daß es sich bei pEBK620-tragenden Keimen tatsächlich um Bakterienklone handelt, die imstande sind, ein atoxisches, aber immunologisch aktives Enterotoxoid zu synthetisieren, wird ein in vitro-Toxizitätstest an Y1-Zellen durchgeführt. Neben Choleratoxin als positiver und plasmidfreien E.coli Keimen als negativer Kontrolle wird der Wildtyp- Enterotoxinproduzentenstamm E.coli 711/P307 neben den durch in vitro- Genrekomination erhaltenen Stämmen E.coli K12 5K/pEFK295 und E.coli K12 5K/pEFK620 eingesetzt.

Die Auswertung des Y1-Tests ergibt, daß nur P307 bzw. pEFK295 tragende Keime die für Enterotoxineinwirkung charakteristischen morphologischen Veränderungen ergeben. Die mit E.coli K12 5K/pEFK620 behandelten Y1-Zellen zeigen keine morphologischen Veränderungen.

6.9     Transformation von Haftantigen-synthetisierenden Bakterien mit dem Enterotoxoid-Plasmid pEFK620

Für eine Vakzine ist neben dem von pEFK620 kodierten Toxoid die Biosynthese eines Haftantigens erforderlich. Die Herstellung solcher Bakterien gelingt durch Transformation von E.coli K12 5K Zellen, die bereits für ein schweinespezifisches K88-Antigen kodierende Plasmide tragen, mit dem Plasmid pEFK620. E.coli K12 Stämme, die porcines Haftantigen der Serotypen K88AB, K88AC und K88AD produzieren, stammen von P. Guinee, National Institute of Health, Bilthoven, Niederlande. Die Transformation dieser Stämme mit je 1 µg des Plasmids pEFK620 erfolgt auf die unter 2.4 beschriebene Weise. Nach erfolgter Transformation von K88 porcinem Haftantigen der Serotypen K88AB bzw. K88AC bzw. K88AD synthetisierenden Keimen mit pEFK620 durchgeführten Plasmid-DNA-Präparationen und entsprechende Restriktionsanalysen zeigen, daß beide Plasmide innerhalb jeweils einer Bakterienzelle stabil sind. Diese Stabilität bleibt auch nach einigen Passagen (mehr als 250 Generationszeiten) in antibiotikafreiem Medium erhalten.

Die Produktion des Enterotoxoids in Anwesenheit des entsprechenden K88-Plasmids wird im passiven Hämolysetest, bestätigt. Auch die Synthese des K88-Antigens wird durch die gleichzeitige Anwesenheit von pEFK295 oder von pEBK620 in der Zelle nicht beeinflußt, wie im Hämagglutinationstest gezeigt werden kann. Der Hämagglutinationstest in Gegenwart von Mannose wird wie beschrieben durchgeführt.

SR/hz

## ABBILDUNG 1

Genkarte des Vektors pBR325, der zu den DNA-Rekombinationsversuchen verwendet wurde.

$Ap^r$, $Cm^r$, $Tc^r$: Lage der Gene für die Resistenzmerkmale gegen die Antibiotika Ampicillin, Chloramphenicol und Tetracyclin.

EcoRI, BamHI: Spaltstellen für die Restriktionsendonukleasen EcoRI und BamHI.

ori: Replikationsursprung

Die Zahlen geben die Größe des Plasmids bzw. der entsprechenden Spaltstücke in Megadalton an.

## ABBILDUNG 2

Genkarte des rekombinanten Plasmids pEFK295.

pEFK 295
[9.4 Md]

EcoRI

BamHI

BamHI

EcoRI

Cm$^r$

Ap$^r$

ori

|  | pBR 325 |
|---|---|
|  | P 307 |
|  | Ent LT (P307) |

EntLT: Gen für hitzelabiles Enterotoxin

P: Promotorregion
A: Gen für die toxische Untereinheit
B: Gen für die bindende Untereinheit restliche Symbole wie in Abb. 1.

## ABBILDUNG 3

Genkarte des rekombinanten Plasmids pEFK500.

$P_{cat}$: Promotor der Chloramphenicolacetyltransferase.

restliche Symbole wie in Abb. 1.

ABBILDUNG 4

ABBILDUNG 5

Genkarte des Vektors pHUB2, der zur Herstellung der rekombinanten Plasmide pEFK700 und pEFK800 verwendet wurde.

Km$^r$, Tc$^r$: Lage der Gene für die Resistenzmerkmale gegen die Antibiotika Kanamycin und Tetracyclin.

EcoRI, BamHI: Spaltstellen für die Restriktionsendonukleasen EcoRI und BamHI.

ori: Replikationsursprung

P$_L$: Lage und Transkriptionsrichtung des vom Bakteriophagen abgeleiteten P$_L$-Promotors.

Die Zahlen geben die Größe des Plasmids bzw. der entsprechenden Spaltstücke in Megadalton an.

ABBILDUNG 6

Genkarte des rekombinanten Plasmids pEFK700

Ent-LT: Gen für hitzelabiles Enterotoxin

P: Promotorregion

A: Gen für die toxische Untereinheit

B: Gen für die bindende Untereinheit

Restliche Symbole wie in Abb. 5

ABBILDUNG 7

Genkarte des rekombinanten Plasmids pEFK800

1.85

Bam H I

Eco R I

Ent LT B

P_L

pEFK 800
[5.9 Md]

Km^r

4.05

ori

| | |
|---|---|
| ▬▬▬▬ | pHUB 2 |
| ▪ ▪ ▪ ▪ ▪ | P 307 |
| ●●●●●●● | Ent LT (P 307) |

Symbole wie in Abb. 5 und 6.

## ABBILDUNG 8

pEFK 295

pKS 100

pEBK 620

| | pBR 325 |
| | P 307 |
| | Ent LT |

Hind III

EcoR I

| | pBR 322 |
| | E.coli chromosomale DNA |

Abbildung 8: Strukturen der Plasmide pEFK295, pKS100 und pEBK620

Nur die für die Konstruktion von pEBK620 wichtigen Restriktionsstellen sind eingezeichnet. Die Abstände zwischen den Restriktionsstellen sind nicht maßstabgerecht.

$Cm^r$, $Ap^r$, Gal symbolisieren die Gene für Chloramphenicolresistenz, Ampicillinresistenz und Galaktosefermentation; P, O, E, T, K stehen für Promotor, Operator, Emiperasegen, Transferasegen und Kinasegen des Galaktoseoperons von E.coli. Ent-LT-B ist das Gen für die B-Untereinheit des E.coli Enteroxins. Die Pfeile über dem Ent-LT-Gen in pEBK620 geben die Transkriptionsrichtung und die mußmaßlichen zwei Startpunkte der Transkription an. Die genauen Koordinaten für die PstI-Erkennungsstelle und die beiden flankierenden HindIII-Sequenzen im P307-Anteil von pEFK295 sind nicht bekannt, ebensowenig wurden die zwei HindIII-Stellen in P307 bestimmt, die das 111 bp lange HindIII-Fragment (Abb. 9) einrahmen. pKS100 ist eine Rekombinante aus pBR322 und chromosomaler DNA aus E.coli. Vom ursprünglichen Plasmid pBR322 wurden die Sequenzen zwischen der EcoRI-Stelle (Pos.4360) und der HindII-Stelle (Pos.652) durch eine ca. 4000 bp lange chromosomale Sequenz ersetzt, die das Gal-Operon enthält.

900-9329/WA

- 69 -

ABBILDUNG 9

◆◆◆◆◆◆◆  gal - operon DNA aus E.coli K12

ⅡⅡⅡⅡⅡⅡ  DNA aus P307

ⓔⓔⓔⓔⓔⓔⓔ  Ent LT-B DNA

gal E

Hind Ⅱ
117
Hind Ⅲ
44
Pst I
152
Hind Ⅲ
111
44
Hind Ⅲ
Pst I
152
Hind Ⅲ
170
15
Hind Ⅱ
EcoR I
235
Ava I
10
Hind Ⅱ
350
Hind Ⅲ

Ent LT-B

gal E

B

gal E

Hind Ⅲ
196
Hind Ⅲ
111
Hind Ⅲ
196
Hind Ⅲ

780

Hind Ⅲ

Ent LT-B

gal E

A

Abbildung 9:

A:   HindIII-Restriktionskarte der DNA aus pEBK620 im Bereich der Integration des LT-B-Gens. Die Pfeile geben die Integrationsrichtung der beiden identischen 196 bp langen HindIII-Fragmente aus dem P307-Anteil von von pEFK295 an.

B:   HindIII, HindII, PstI, EcoRI und AvaI Restriktionskarte der DNA aus pEBK620 im Bereich der Integration des Ent-LT-B-Gens.

Die Karten in A und B sind maßstabgerecht. Die Zahlen unter den Karten geben die DNA-Längen in Basenpaaren (bp) an.

## ABBILDUNG 10

gal E

Hind II

Hind III
Pst I

Hae III
Hind III

Hind III
Pst I

Hae III
Hind III

Hind II
EcoR I

Ava I
Hind II

Hind III

gal E

Ent LT-B

◆◇◆◇◆◆◇   gal - operon DNA aus E.coli K12

ⁿⁿⁿⁿⁿⁿ   DNA aus P307

⊕⊕⊕⊕⊕⊕⊕   Ent LT-B DNA

Abbilung 10: Sequenzierungsstrategie für das 795 bp lange HindII-Fragment aus pEBK620

Das 795 bp HindII-Fragment wird mit der Restriktionsendonuklease PstI gespalten und mit Phosphatase behandelt. Nach Reinigung der DNA an Sephadex G100 werden die DNA-Stücke an ihrem 5'-Ende mit Polynukleotidkinase und $\gamma$-$^{32}$P radioaktiv markiert und in einem 1. Experiment mit der Restriktionsendonuklease HaeIII, in einem 2. Experiment mit der Restriktionsendonuklease HindIII nachgespalten. Die so gewonnenen radioaktiven Spaltprodukte werden in einem 12 %igen Polyacrylamidgel elektrophoretisch aufgetrennt und nach Autoradiographie des Gels ausgeschnitten und eluiert. Sequenziert wird nach Maxam und Gilbert. Die sequenzierten Teile der DNA sind mit Pfeilen in Abb. 10 gekennzeichnet.

## ABBILDUNG 11

Struktur der pBR322-Derivate mit den subklonierten 111 bp bzw. 196 bp HindIII-Fragmenten. Die Pfeilspitze innerhalb der Plasmidstruktur gibt jeweils die Integrationsrichtung der subklonierten Banden an. Die Pfeile darunter zeigen an, welcher Teil der DNA sequenziert wurde.

ABBILDUNG   12

→ gal E

5′ GACGGCACTC   TGCGCCTGAT   TAGCGCCATG   CGGCCCGCTA   ACGTCAAAAA   CTTTATTTTT   AGCTCCTCCG
   CTGCCGTGAG   ACGCGGACTA   ATCGCGGTAC   GCCGGGCGAT   TGCAGTTTTT   GAAATAAAAA   TCGAGGAGGC
H2                                       Ha3                      → 196 bp

   CCACCGTTTA   TGGCGATCAG   CCCAAAATTC   CATACGTTGA   AANNNAGCTT   CGGAAAGCCG   TATCGTGGGT
   GGTGGCAAAT   ACCGCTAGTC   GGGTTTTAAG   GTATGCAACT   TTNNTTCGAA   GCCTTTCGGC   ATAGCACCCA
                                                      H3

   ATCGCTCAGC   CCCAGCCTGC   AGGGCGGCAA   TAACGGGTTC   ATCACGCGTG   GTATCCGGGC   CGTAATGCTA
   TAGCGAGTCG   GGGTCGGACG   TCCCGCCGTT   ATTGCCCAAG   TAGTGCGCAC   CATAGGCCCG   GCCATTACGAT
                P1

   AACCGTTCTG   CTCAGGTTCA   GACTCCGGCA   GGCCTGACGG   ATACTGAGTC   CGAAATGTCG   TTATCAGATG
   TTGGCAAGAC   GAGTCCAAGT   CTGAGGCCGT   CCGGACTGCC   TATGACTCAG   GCTTTACAGC   AATAGTCTAC
                                        Ha3 → 111bp

   AGTGCCCCGC   TCACGTTAAA   AGCTGATTTT   AAGCTTCTGA   TATTAAAAAA   ATAAGGATC   TTGAGGACGA
   TCACGGGGCG   AGTGCAATTT   TCGACTAAAA   TTCGAAGACT   ATAATTTTTT   TATTTCCTAG   AACTCCTGCT
                                        H3

   GAACCGCCGT   CTCAAACAGA   TGTTTGCTGA   CCTGAGTCTG   GAAAACCGGG   CGCTGAAAGA   CGTTATCGAA
   CTTGGCGGCA   GAGTTTGTCT   ACAAACGACT   GGACTCAGAC   CTTTTGGCCC   GCGACTTTCT   GCAATAGCTT

      → 196bp
   AAAAGCTTCG   GAAAGCCGTA   TCGTGGGTAT   CGCTCAGCCGC   AGCCTGCAGG   GCGGCAATAA   CGGGTTCATC
   TTTTCGAAGC   CTTTCGGCAT   AGCACCCATA   GCGAGTCGGCG   TCGGACGTCC   CGCCGTTATT   GCCCAAGTAG
   H3                                                 P1

   ACGCGTGGTA   TCCGGGCGGT   AATGGTAAAC   CGTTCTGCTC   AGGTTCAGAC   TCCGGCAGGC   CTGACGGATA
   TGCGCACCAT   AGGCCCGCCA   TTACCATTTG   GCAAGACGAG   TCCAAGTCTG   AGGCCGTCCG   GACTGCCTAT
                                                                                Ha3 → Ent − LT

   CTGAGTCCGA   AATGTCGTTA   TCAGATGAGT   GCCCCGCTCA   CGTTAAAAGC   TGATTTTAAG   CTTNNNNNNN
   GACTCAGGCT   TTACAGCAAT   AGTCTACTCA   CGGGGCGAGT   GCAATTTTCG   ACTAAAATTC   GAANNNNNNN
                                                                                H3

   NNNNNNNNNN   NNNNNNNNNN   NNNNNNNNNN   CAGGAAATTC   AATCAAGAAC   AATCAGAGGT   GTACTTGTAA
   NNNNNNNNNN   NNNNNNNNNN   NNNNNNNNNN   GTCCTTTAAG   TTAGTTCTTG   TTAGTCTCCA   CATGAACATT

   TGAGGAGACC   CAGAATCTGA   GCACAATATA   TCTCAGGGAA   TATCAATCAA   AAGTTAAGAG   GCAGATATTT
   ACTCCTCTGG   GTCTTAGACT   CGTGTTATAT   AGAGTCCCTT   ATAGTTAGTT   TTCAATTCTC   CGTCTATAAA

   TCAGACTATC   AGCTCAAGGT   PyPuAC
   ATTCTGATAG   TCGAGTTCCA   PuPyTG   5′
                            H2

## ABBILDUNG 12

**DNA-Sequenz des 795 bp langen HindII-Fragments aus pEBK620**

Die Sequenz beginnt an der HindII-Erkennungsstelle in galE und endet an der HindII-Erkennungsstelle im Ent-LT-A-Gen. Die mit einer geschlängelten Linie gekennzeichneten Sequenzen stellen Restriktionserkennungsstellen dar, und zwar für die Restriktionsenzyme HindII = H2, HindIII = H3, HaeIII = Ha3, PstI = P1.

C = Cydithylsäure, G = Guanylsäure, T = Thimidylsäure, A = Adenylsäure, N = Sequenz der Basen, in der DNA nicht bekannt ist.

## TABELLE 5

Durchschnittliche Enterotoxin (-toxoid)-Ausbeuten (passive Immunhämolyse) und Ergebnisse des $Y_1$-Zelltests[1]

| Keim: | Plasmid(e) | Ent-LT(Ü)[2] [µg/l] | Ent-LT(Z)[3] [µg/l] | Entero-. toxin | Herkunft: | $Y_1$-Test |
|-------|-----------|---------------------|---------------------|----------------|-----------|------------|
| E.coli 711 | P307 | $10^{4)}$ | 25 ± 40% | Toxin | Wildtyp-Porcin | pos. |
| E.coli K12 5K | pEFK700 pRK248cIts | 900 | 1900 ± 10% | Toxin | pEFK295$\xrightarrow{[BamHI]}$pEFK700 pHUB2 | pos. |
| E.coli K12 5K | pEFK800 + pRK248cIts | 5500 ± 20% | 560 ± 10% | Toxoid | pEFK700$\xrightarrow{[EcoRI]}$pEFK800 | neg. |

1) Die angeführten Ergebnisse beziehen sich auf Keime, die unter den beschriebenen Labor-Züchtungsbedingungen in Schüttelkultur erhalten wurden (P307 tragende Keime bei 37° C, rekombinante Klone bei 42° C; kein Antibiotikazusatz).

2) Enterotoxin (-toxoid)-Konzentration im Kulturüberstand nach Abzentrifugation des Zellmaterials, angegeben in µg Choleratoxin-Äquivalenten pro Liter Kulturbrühe.

3) Intrazelluläre Enterotoxin (-toxoid)-Menge aus zentrifugiertem, ultrabeschalltem Zellmaterial, angegeben in µg Choleratoxin-Äquivalenten pro Zellmasse, gewonnen aus einem Liter Kulturbrühe.

4) Die Nachweisgrenze des unter den beschriebenen Bedingungen durchgeführten passiven Immunhämolysetests liegt bei etwa 10 µg VCT-Äquivalenten/Liter. In den mit 10 bezeichneten Fällen konnten im Immunhämolysetest keine meßbaren Hämolyschöfe nachgewiesen werden.

## TABELLE 6

Abhängigkeit der Enterotoxoidsynthese des Stammes E. coli K12 5K/pEFK800 + pRK248clts + K88 (AB) von den Züchtungsbedingungen[1)]

| Keim: | Züchtungsbedingung | µg Ent-LT/l(Ü) | µg Ent-LT/l(Z) | Gesamt: | % im Überstand. |
|---|---|---|---|---|---|
| E. coli K12 | 30°C (-AB)[2)] | 394 | 77 | 471 | 81 |
| 5K/peFK800 + | 30°C (+AB)[3)] | 865 | 203 | 1068 | 83 |
| pRK248clts + | 37°C (-AB)[4)] | 1041 | 163 | 1204 | 86 |
| K88 (AB) | 37°C (+AB)[5)] | 1120 | 600 | 1720 | 66 |
| | 30°C (+AB) 45°C 42°C (-AB)[6)] | 1300 | 5700 | 7000 | 19 |
| | 30°C (-AB) 45°C 42°C (-AB)[7)] | 1900 | 1800 | 3700 | 52 |
| | 42°C (-AB)[8)] | 5280 | 530 | 5810 | 91 |

1) Im Hybridplasmid pEFK800 steht das Ent-LT (B) Gen unter der Kontrolle des vom -Phagen stammenden $P_L$-Promotors, der seinerseits von dem vom Plasmid pRK248clts kodierten cI-Repressor reguliert wird. Aus der Temperatursensitivität des cI-Repressorgens erklären sich die stark unterschiedlichen Ent-LT (B)-Ausbeuten bei verschiedenen Temperaturen. Die Angaben für die Kulturüberstände (Ü) bzw. Zellysate (Z) beziehen sich auf µg Choleratoxin-Äquivalente pro Liter Kulturbrühe, gemessen im passiven Immunhämolysetest.

2) Züchtung bei 30° C ohne Antibiotikumszusatz (Kanamycin).

3) Züchtung bei 30° C mit Antibiotikumszusatz (50 µg Kanamycin/ml Medium).

4) Züchtung bei 37° C ohne Antibiotikumszusatz

5) Züchtung bei 37° C mit Antibiotikumszusatz (50 µg Kanamycin/ml Medium).

6) Anzucht der Zellen bei 30° C (12 Stunden) mit Antibiotikum (50µg Kanamycin/ml Medium), Zentrifugation, Aufnahme der Zellmasse in Nährmedium ohne Antibiotikum, kurzes Bebrüten bei 45° C ( 30 Minuten), Senken der Temperatur auf 42° C und Inkubation bei 42° C (4 Stunden).

7) Anzucht der Zellen bei 30° C (12 Stunden) ohne Antibiotikum, Erhöhung der Temperatur auf 45° C, kurzes Bebrüten ( 30 Minuten), Senken der Temperatur auf 42° C und Inkubation bei 42° C (4 Stunden).

8) Züchtung bei 42° C ohne Antibiotikumszusatz.

SR/hz

Patentansprüche

1. Verfahren zur Herstellung des Plasmids pEFK600, dadurch gekennzeichnet, daß man das Plasmid pEFK 295 enzymatisch mit der Restriktionsendonuklease HindIII öffnet, die entstandenen Fragmente mit T4-Ligase ligiert, nach Transformation kompetenter E. coli K12 5K-Zellen mit der im Ligasegemisch anwesenden DNA die ampicillin- und chloramphenicolresistenten Transformanten selektioniert, mit Hilfe des passiven Immunhämolysetests sowie des Y1-Zelltests jene Klone indentifiziert, die immunologisch aktives, aber nicht toxisches Enterotoxoid synthetisieren und aus den ausgewählten Klonen jenes rekombinante Plasmid isoliert, das eine vom Ent-Plasmid P307 stammende Genregion von 1,85 Md (2,8 kb) enthält.

2. Verfahren zur Herstellung des Plasmids pEFK700, dadurch gekennzeichnet, daß man das Plasmid P307 sowie den Plasmidvektor pHUB2 mit der Restriktionsnuklease BamHI enzymatisch öffnet, die entstandenen Fragmente mit T4-Ligase ligiert, nach Transformation kompetenter E.coli K12 5K Zellen, welche das Plasmid pRK248cIts beherbergen, mit der im Ligasegemisch anwesenden DNA die tetracyclin- und kanamycinresistenten Transformanten selektioniert und aus den ausgewählten Klonen jenes rekombinante Plasmid isoliert, das ein vom Ent-Plasmid P307 stammendes BamHI-Restriktionsfragment von 5,8 Md (8,78 kb) enthält.

3. Verfahren zur Herstellung des Plasmids pEFK800, dadurch gekennzeichnet, daß man das Plasmid pEFK700 mit der Restriktionsendonuklease EcoRI enzymatisch öffnet, anschließend das größere der dabei entstandenen Fragmente durch Ligierung mit T4-Ligase cyclisiert, nach Transformation kompetenter E.coli K12 5K/pRK248cIts Zellen mit der im Ligasegemisch anwesenden DNA jene Transformanten, die Tetracyclin- und Kanamycinresistenz aufweisen, selektioniert und das Plasmid pEFK800 isoliert.

4. Verfahren zur Herstellung des Plasmids pEBK620, dadurch gekennzeichnet, daß man das Plasmid pEFK295 mit der Restriktionsendonuklease HindIII spaltet sowie den Plasmidvektor pKS100 mit der Restriktionsendonuklease HindIII enzymatisch öffnet und mit alkalischer Phosphatase behandelt, die entstandenen Fragmente mit T4-Ligase ligiert, nach Transformation kompetenter E.coli K12 F 165Δ(gal) trp-Zellen mit der im Ligasegemisch anwesenden DNA die ampicillinresistenten Transformanten selektioniert und aus den ausgewählten Klonen jenes rekombinante Plasmid isoliert, das vier vom Ent-Plasmid pEFK295 stammende HindIII-Restriktionsfragmente mit Längen von 111 Basenpaaren (bp), 196 bp, 196 bp und 780 bp enthält.

5. Ein Plasmid ausgewählt aus pEFK600, -700, -800 und pEBK620.

6. Verfahren zur Herstellung des Enterotoxoid-produzierenden Stammes E. coli K12 5K/pEFK600, dadurch gekennzeichnet, daß man den Wirtsstamm E. coli K12 5K mit dem Plasmid pEFK600 transformiert und jene Transformanten selektioniert, die ampicillin- und chloramphenicolresistent sind.

7. Verfahren zur Herstellung des Enterotoxin-produzierenden Stammes E. coli K12 5K/pEFK700 + pRK248cIts, dadurch gekennzeichnet, daß man den Wirtsstamm E.coli K12 5K mit den Plasmiden pRK248cIts und pEFK700 transformiert und jene Transformanten selektioniert, die tetracyclin- und kanamycinresistent sind.

8. Verfahren zur Herstellung des Enterotoxoid-produzierenden Bakterienstammes E.coli K12 5K/pEFK800 + pRK248cIts, dadurch gekennzeichnet, daß man den Wirtsstamm E.coli K12 5K mit den Plasmiden pRK248cIts und pEFK800 transformiert und jene Transformanten selektioniert, die Tetracyclin- und Kanamycinresistenz aufweisen.

**0084522**

9. Verfahren zur Herstellung des Enterotoxoid-produzierenden Stammes E.coli K12 5K/pEBK620, dadurch gekennzeichnet, daß man den Wirtsstamm E.coli K12 5K mit dem Plasmid pEBK620 transformiert und jene Transformanten selektioniert, die ampicillinresistent sind.

10. Verfahren zur Herstellung des Enterotoxoid- und K88-Antigen-produzierenden Bakterienstammes E. coli K12 5K/pEFK600 + K88(AB), dadurch gekennzeichnet, daß man K88-Haftantigen-produzierende Keime des Serotyps AB mit dem Plasmid pEFK600 transformiert und jene Transformanten selektioniert, die Ampicillin- und Chloramphenicolresistenz aufweisen.

11. Verfahren zur Herstellung des Enterotoxin- und K88-Antigen-produzierenden Bakterienstammes E.coli K12 5K/pEFK700 + pRK248cIts + K88(AB), dadurch gekennzeichnet, daß man K88-Haftantigen-produzierende Keime des Serotyps AB mit den Plasmiden pRK248cIts und pEFK700 transformiert und jene Transformanten selektioniert, die Tetracyclin- und Kanamycinresistenz aufweisen.

12. Verfahren zur Herstellung des Enterotoxoid- und K88-Antigen-produzierenden Bakterienstammes E.coli K12 5K/pEFK800 + pRK248cIts + K88(AB), dadurch gekennzeichnet, daß man K88-Haftantigen-produzierende Keime des Serotyps AB mit den Plasmiden pRK248cIts und pEFK800 transformiert und jene Transformanten selektioniert, die Tetracyclin- und Kanamycinresistenz aufweisen.

13. Verfahren zur Herstellung der Enterotoxoid- und K88-Antigen-produzierenden Bakterienstämme E. coli K12 5K/pEBK620 + K88(AB, AC, AD), dadurch gekennzeichnet, daß man K88-Haftantigen-produzierende Keime des Serotyps AB, AC, AD mit dem Plasmid pEBK620 transformiert und jene Transformanten selektioniert, die Ampicillinresistenz aufweisen.

14. Ein Bakterienstamm ausgewählt aus:

Enterotoxoid produzierendes E.coli K12 5K/pEFK600, E.coli K12 5K/pEFK800 + pRK248cIts und E.coli K12 5K/pEBK620; Enterotoxin produzierendes E.coli K12 5K/pEFK700 + pRK248cIts; Enterotoxoid- und K88-Antigen-produzierendes E.coli K12 5K pEFK600 + K88(AB), E.coli K12 K/pEFK800 + pRK248cIts + K88(AB) und E.coli K12 5K/pEBK620 + K88 (AB, AC, AD); sowie Enterotoxin- und K88-Antigen-produzierendes E.coli K12 5K/pEFK700 + pRK248cIts + K88(AB).

15. Alle hierin beschriebenen neuen Erfindungsmerkmale.